(19) Europäisches Patentamt
European Patent Office
Office européen des brevets

(11) Publication number: **0 416 393 A1**

(12) **EUROPEAN PATENT APPLICATION**

(21) Application number: **90116225.5**

(22) Date of filing: **24.08.90**

(51) Int. Cl.5: **C07K 5/06**, C07D 233/64, C07D 277/30, A61K 31/415, A61K 31/54, A61K 37/64

(30) Priority: **05.09.89 US 403437**
**06.08.90 US 561537**

(43) Date of publication of application:
**13.03.91 Bulletin 91/11**

(84) Designated Contracting States:
**AT BE CH DE DK ES FR GB GR IT LI LU NL SE**

(71) Applicant: **ABBOTT LABORATORIES**
**CHAD-0377, AP6D/2, One Abbott Park Road**
**Abbott Park, Illinois 60064-3500(US)**

(72) Inventor: **Sham, Hing L.**
**5109 Pembrook Court**
**Gurnee, Illinois 60031(US)**
Inventor: **Rosenberg, Saul H.**
**1110 Fairlawn Drive**
**Libertyville, Illinois 60048(US)**

(74) Representative: **Modiano, Guido et al**
**MODIANO, JOSIF, PISANTY & STAUB**
**Modiano & Associati Via Meravigli, 16**
**I-20123 Milano(IT)**

(54) **Peptidyl difluorodiol renin inhibitors.**

(57) A renin inhibiting compound of the formula:

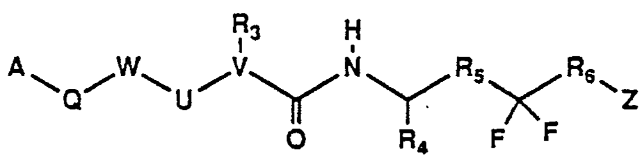

wherein A is a functional group;
W is
(1) -C(O)-,
(2) -CH(OH)- or
(3) $-N(R_2)-$ wherein $R_2$ is hydrogen or loweralkyl; U is
(1) -C(O)-,
(2) $-CH_2-$ or
(3) $-N(R_2)-$ wherein $R_2$ is hydrogen or lower alkyl, with the proviso that when W is -CH(OH)-then U is $-CH_2-$ and with the proviso that U is -C(O)- or $-CH_2-$ when W is $-N(R_2)-$; V is
(1) -CH-,
(2) -C(OH)- or
(3) -C(halogen)- with the proviso that v is -CH-when U is $-N(R_2)-$; Q is $-CH(R_1)-$ or $-C(=CHR_{1a})-$ wherein $R_1$ is
(1) loweralkyl,
(2) cycloalkylalkyl,
(3) arylalkyl,
(4) (heterocyclic)alkyl,
(5) 1-benzyloxyethyl,
(6) phenoxy,

EP 0 416 393 A1

(7) thiophenoxy or
(8) anilino, provided that B is $-CH_2-$ or -CH(OH)-or A is hydrogen when $R_1$ is phenoxy, thiophenoxy or anilino and $R_{1a}$ is aryl or heterocyclic; $R_3$ is a functional group;
$R_4$ is
(1) loweralkyl,
(2) cycloalkylmethyl or
(3) benzyl; $R_5$ is -CH(OH)- or -C(O)-;
$R_6$ is -CH(OH)- or -C(O)-; and
Z is
(1) lower alkyl,
(2) aryl,
(3) arylalkyl,
(4) cycloalkyl,
(5) cycloalkylalkyl,
(6) heterocyclic or
(7) (heterocyclic)alkyl; or a pharmaceutically acceptable salt, ester or prodrug thereof.

# PEPTIDYL DIFLUORODIOL RENIN INHIBITORS

This is a continuation-in-part of U.S. patent application Serial No. 403,437, filed Septmeber 5, 1989.

## Technical Field

The present invention relates to novel compounds and compositions which inhibit renin, processes for making such compounds, synthetic intermediates employed in these processes and a method of treating hypertension or congestive heart failure with such compounds or in combination with another antihypertensive agent. The present invention also relates to compositions and a method for treating glaucoma, vascular disease, renal failure or psoriasis with such compounds and a method of inhibiting retroviral proteases and treating a retroviral infection with such compounds.

## Background Art

Renin is a proteolytic enzyme synthesized and stored principally in a specific part of the kidney called the juxtaglomerular apparatus. Any of three different physiologic circumstances may cause the release of renin into the circulation; (a) a decrease in the blood pressure entering or within the kidney itself; (b) a decrease in the blood volume in the body; or (c) a fall in the concentration of sodium in the distal tubules of the kidney.

When renin is released into the blood from the kidney, the renin-angiotensin system is activated, leading to vasoconstriction and conservation of sodium, both of which result in increased blood pressure. The renin acts on a circulating protein, angiotensinogen, to cleave out a fragment called angiotensin I (AI). AI itself has only slight pharmacologic activity but, after additional cleavage by a second enzyme, angiotensin converting enzyme (ACE), forms the potent molecule angiotensin II (AII). The major pharmacological effects of AII are vasoconstriction and stimulation of the adrenal cortex to release aldosterone, a hormone which causes sodium retention. Sodium retention causes blood volume to increase, which leads to hypertension. AII is cleaved by an aminopeptidase to form angiotensin III (AIII), which, compared to AII, is a less potent vasoconstrictor but a more potent inducer of aldosterone release.

Inhibitors of renin have been sought as agents for control of hypertension and as diagnostic agents for identification of cases of hypertension due to renin excess.

With these objectives in mind, the renin-angiotensin system has been modulated or manipulated, in the past, with ACE inhibitors. However, ACE acts on several substrates other than angiotensin I (AI), most notably the kinins which cause such undesirable side effects as pain, "leaky" capillaries, prostaglandin release and a variety of behavorial and neurologic effects. Further, ACE inhibition leads to the accumulation of AI. Although AI has much less vasoconstrictor activity than AII, its presence may negate some of the hypotensive effects of the blockade of AII synthesis.

Inhibition of other targets in the renin-angiotensin system such as AII with compounds such as saralasin can block AII activity, but would leave unimpaired and perhaps enhance the hypertensive effects of AIII.

On the other hand, there are no known side effects which result when renin is inhibited from acting on its substrate. Considerable research efforts have thus been carried out to develop useful inhibitors of renin. Past research efforts have been directed to renin antibodies, pepstatin, phospholipids and substrate analogs such as tetrapeptides and octapeptides to tridecapeptides. These inhibitors either demonstrate poor activity in inhibiting renin production or poor specificity for inhibiting renin only. However, Boger et al. have reported that statine-containing peptides possess potent and specific renin-inhibiting activity ( Nature , Vol. 303, p. 81, 1983). In addition, Szelke and co-workers have described polypeptide analogs containing a non-peptide link ( Nature , Vol. 299, p. 555, 1982) which also cause potent renin inhibition and show a high specificity for this enzyme. Recent patents have disclosed novel small peptide renin inhibitors which contain novel dipeptide isosteres as transition state analogs (Szelke, et al., U.S. Patent No. 4,609,643; Boger, et al., U.S. Patent No. 4,668,770; Baran, et al., U.S. Patent No. 4,657,931; Matsueda, et al., U.S. Patent No. 4,548,926; Luly, et al., U.S. Patent No. 4,645,759; and Luly, et al., U.S. Patent No. 4,680,284).

## Disclosure of the Invention

In accordance with the present invention, there are renin inhibiting compounds of the formula:

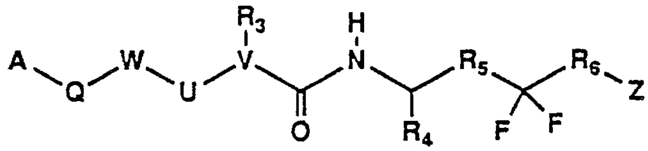

(I)

or a pharmaceutically acceptable salt, ester or prodrug thereof.

A is
(1) hydrogen,
(2) loweralkyl,
(3) functionalized alkyl,
(4) aryl,
(5) heterocyclic,
(6) $-OR_7$ or $-SR_7$ wherein $R_7$ is
(i) hydrogen,
(ii) loweralkyl,
(iii) functionalized alkyl,
(iv) aryl,
(v) heterocyclic,
(vi) aryl-C(O)- or
(vii) heterocyclic-C(O)-,
(7) carboxy,
(8) alkoxycarbonyl,
(9) functionalized amino,
(10) functionalized hydroxyalkyl or
(11) substituted carbonyloxy or substituted carbonyloxy analog.

W is
(1) -C(O)-,
(2) -CH(OH)- or
(3) $-N(R_2)-$ wherein $R_2$ is hydrogen or loweralkyl.

U is
(1) -C.(0)-,
(2) $-CH_2-$ or
(3) $-N(R_2)-$ wherein $R_2$ is hydrogen or lower alkyl, with the proviso that when W is -CH(OH)-then U is $-CH_2-$ and with the proviso that U is -C(O)- or $-CH_2-$ when W is $-N(R_2)-$.

V is
(1) -CH-,
(2) -C(OH)- or -
(3) -C(halogen)- with the proviso that V is -CH-when U is $-N(R_2)-$.

Q is $-CH(R_1)-$ or $-C(=CHR_{1a})-$ wherein $R_1$ is
(1) loweralkyl,
(2) cycloalkylalkyl,
(3) arylalkyl,
(4) (heterocyclic)alkyl,
(5) 1-benzyloxyethyl,
(6) phenoxy,
(7) thiophenoxy or
(8) anilino, provided that B is $-CH_2-$ or -CH(OH)-or A is hydrogen when $R_1$ is phenoxy, thiophenoxy or anilino and $R_{1a}$ is aryl or heterocyclic.

$R_3$ is
(1) loweralkyl,
(2) loweralkenyl,
(3) hydroxyalkyl,
(4) alkoxyalkyl,

(5) ((alkoxy)alkoxy)alkyl,
(6) carboxyalkyl,
(7) (thioalkoxy)alkyl,
(8) azidoalkyl,
(9) aminoalkyl,
(10) (alkyl)aminoalkyl,
(11) (dialkyl)aminoalkyl,
(12) (alkoxy)(alkyl)aminoalkyl,
(13) (alkoxy)aminoalkyl,
(14) benzyl or
(15) (heterocyclic)methyl.

$R_4$ is
(1) loweralkyl,
(2) cycloalkylmethyl or
(3) benzyl.

$R_5$ is
(1) -CH(OH)- or
(2) -C(O)-.

$R_6$ is
(1) -CH(OH)- or
(2) -C(O)-.

Z is
(1) lower alkyl,
(2) aryl,
(3) arylalkyl,
(4) cycloalkyl,
(5) cycloalkylalkyl,
(6) heterocyclic or
(7) (heterocyclic)alkyl.

The compounds of formula I contain two or more asymmetric carbon atoms and thus can exist as pure diastereomers, mixtures of diastereomers, diastereomeric racemates or mixtures of diastereomeric racemates. The present invention includes within its scope all of the isomeric forms. The terms "R" and "S" configuration used herein are as defined by IUPAC 1974 Recommendations for Section E, Fundamental Stereochemistry, Pure Appl. Chem (1976) 45, 13-30.

The term "loweralkyl" as used herein refers to straight or branched chain alkyl radicals containing from 1 to 7 carbon atoms including, but not limited to, methyl, ethyl, n-propyl, iso-propyl, n-butyl, iso-butyl, sec-butyl, n-pentyl, 2-methylbutyl, 2,2-dimethylpropyl, n-hexyl, 2-methylpentyl, 2,2-dimethylbutyl, n-heptyl and the like.

The term "functionalized alkyl" as used herein includes:
(1) aminoalkyl,
(2) (alkyl)aminoalkyl,
(3) (dialkyl)aminoalkyl,
(4) (alkoxy)aminoalkyl,
(5) (alkoxy)(alkyl)aminoalkyl,
(6) arylalkyl and
(7) (heterocyclic)alkyl.

The term "cycloalkyl" as used herein refers to an alicyclic ring having 3 to 7 carbon atoms including, but not limited to, cyclopropyl, cyclopentyl, cyclohexyl and the like.

The term "cycloalkylalkyl" as used herein refers to a cycloalkyl residue appended to a loweralkyl radical and includes, but is not limited to, cyclohexylmethyl and cyclopentylmethyl.

The term "loweralkenyl" as used herein refers to a straight or branched chain of 2 to 7 carbon atoms having at least one carbon-carbon double bond including, but not limited to, vinyl, propenyl, butenyl and the like.

The term "arylalkyl" as used herein refers to a aryl group appended to a loweralkyl radical, including, but not limited to benzyl, phenethyl, 1-naphthylmethyl, 2-naphthylmethyl and the like.

The term "(heterocyclic)alkyl" as used herein refers to an unsubstituted or substituted heterocyclic ring as defined herein appended to a lower alkyl radical, including, but not limited to imidazolylmethyl, thiazolylmethyl and the like.

The term "(heterocyclic)methyl" as used herein refers to a heterocyclic group appended to a methyl radical.

The term "hydroxyalkyl" as used herein refers to -OH appended to a loweralkyl radical.

The term "alkoxyalkyl" as used herein refers to an alkoxy group appended to a loweralkyl radical.

The term "thioalkoxyalkyl" as used herein refers to a thioalkoxy group appended to a loweralkyl radical.

The term "arylalkoxyalkyl" as used herein refers to an arylalkoxy group appended to a loweralkyl radical, including, but not limited to phenylmethoxymethyl, naphthylmethoxymethyl and the like.

The term "((alkoxy)alkoxy)alkyl" as used herein refers to an alkoxy group appended to an alkoxy group which is appended to a loweralkyl radical, including, but not limited to methoxymethoxymethyl and the like.

The term "(polyalkoxy)alkyl" as used herein refers to a polyalkoxy residue appended to a loweralkyl radical, including, but not limited to methoxyethoxymethoxymethyl and the like.

The term "aminoalkyl" as used herein refers to $-NH_2$ appended to a loweralkyl radical.

The term "(alkyl)aminoalkyl" as used herein refers to $-NHR_{12}$ appended to a loweralkyl radical, wherein $R_{12}$ is a loweralkyl radical.

The term "(dialkyl)aminoalkyl" as used herein refers to a dialkylamino group appended to a loweralkyl radical.

The term "aminocycloalkyl" as used herein refers to an $-NH_2$ appended to a cycloalkyl radical.

The term "(N-protected)aminoalkyl" as used herein refers to $-NHR_{13}$ appended to a loweralkyl group, wherein $R_{13}$ is an N-protecting group.

The term "(N-protected)(alkyl)aminoalkyl" as used herein refers to $-NR_{13}R_{14}$, which is appended to a loweralkyl radical, wherein $R_{13}$ is defined as above and $R_{14}$ is a loweralkyl group.

The term "alkoxycarbonylalkyl" as used herein refers to $R_{15}COR_{16}$-, wherein $R_{15}$ is an alkoxy group and $R_{16}$ is a loweralkyl radical.

The term "carboxyalkyl" as used herein refers to a carboxylic acid group (-COOH) appended to a loweralkyl radical.

The term "cyanoalkyl" as used herein refers to -CN appended to a loweralkyl radical.

The term "(alkoxy)aminoalkyl" as used herein refers to an alkoxy group appended to an amino group which in turn is appended to a loweralkyl radical.

The term "(alkoxy)(alkyl)aminoalkyl" as used herein refers to an $-NR_{59}R_{60}$ group appended to a loweralkyl radical wherein $R_{59}$ is an alkoxy group and $R_{60}$ is a loweralkyl group.

The term "haloalkyl" as used herein refers to a loweralkyl radical substituted one or more halogens including, but not limited to, fluoromethyl, 2-chloroethyl, trifluoromethyl, 2,2-dichloroethyl and the like.

The term "azidoalkyl" as used herein refers to a $-N_3$ group appended to a loweralkyl radical.

The term "loweralkylsulfinylalkyl" as used herein refers to a $R_{61}S(O)$- group appended to a loweralkyl radical wherein $R_{61}$ is a loweralkyl group.

The term "loweralkylsulfonylalkyl" as used herein refers to a $R_{62}S(O)_2$- group appended to a loweralkyl radical wherein $R_{62}$ is a loweralkyl group.

The term "arylthioalkyl" as used herein refers to a $R_{63}S$- group appended to a loweralkyl radical wherein $R_{63}$ is an aryl group.

The term "arylsulfonylalkyl" as used herein refers to a $R_{67}S(O)_2$- group appended to a loweralkyl radical wherein $R_{67}$ is an aryl group.

The term "functionalized hydroxyalkyl" as used herein includes $R_{41}CH(OH)CH_2$- and $R_{41}CH(OH)CH(OH)$-
wherein $R_{41}$ is
(i) loweralkyl,
(ii) cycloalkyl,
(iii) aryl,
(iv) arylalkyl,
(v) arylalkoxyalkyl,
(vi) thioalkoxyalkyl,
(vii) loweralkylsulfinylalkyl,
(viii) loweralkylsulfonylalkyl,
(ix) arylthioalkyl,
(x) arylsulfonylalkyl,
(xi) aminoalkyl,
(xii) alkoxycarbonylalkyl,
(xiii) carboxyalkyl,
(xiv) (N-protected)aminoalkyl,

(xv) (alkyl)aminoalkyl,
(xvi) (N-protected)(alkyl)aminoalkyl,
(xvii) (dialkyl)aminoalkyl,
(xviii) (heterocyclic)alkyl,
(xix) heterocyclic,
(xx) aminocycloalkyl or
(xxi) (polyalkoxy)alkyl.

The term "amino" as used herein refers to an $-NH_2$ substituent.

The term "functionalized amino" as used herein includes $-NR_8R_9$ wherein $R_8$ and $R_9$ are independently selected from:
(i) hydrogen,
(ii) hydroxy,
(iii) alkoxy,
(iv) loweralkyl,
(v) aminoalkyl,
(vi) cyanoalkyl and
(vii) hydroxyalkyl.

The term "alkylamino" as used herein refers to $-NHR_{17}$, wherein $R_{17}$ is a loweralkyl group.

The term "dialkylamino" as used herein refers to $-NR_{18}R_{19}$, wherein $R_{18}$ and $R_{19}$ are independently selected from loweralkyl groups.

The term "arylalkylamino" as used herein refers to a arylalkyl group appended to an amino radical including, but not limited to, benzylamino and the like.

The term "(arylalkyl)(alkyl)amino" as used herein refers to $R_{21}R_{22}N$-, wherein $R_{21}$ is an arylalkyl residue and $R_{22}$ is a loweralkyl residue.

The term "aminoalkylamino" as used herein refers to $R_{23}NH$-, where $R_{23}$ is an aminoalkyl residue.

The term "(dialkylaminoalkyl)(alkyl)amino" as used herein refers to $R_{24}R_{25}N$- wherein $R_{24}$ is a dialkylamino residue appended to a loweralkyl group and $R_{25}$ is a loweralkyl group.

The term "(hydroxyalkyl)(alkyl)amino" as used herein refers to $-NR_{26}R_{27}$ wherein $R_{26}$ is a hydroxyalkyl group and $R_{27}$ is a loweralkyl group.

The term "(di-hydroxyalkyl)(alkyl)amino" as used herein refers to a loweralkyl group which is disubstituted with -OH radicals appended to an amino group, which amino group also has appended another loweralkyl group.

The term "di-(hydroxyalkyl)amino" as used herein refers to $R_{28}R_{29}N$-, wherein $R_{28}$ and $R_{29}$ are hydroxyalkyl residues.

The term "(alkoxyalkyl)(alkyl)amino" as used herein refers to $R_{30}R_{31}N$-, wherein $R_{30}$ is an alkoxyalkyl group and $R_{31}$ is a loweralkyl group.

The term "di-(alkoxyalkyl)amino" as used herein refers to $R_{32}R_{33}N$-, wherein $R_{32}$ and $R_{33}$ are alkoxy groups appended to a loweralkyl residue.

The term "di-(polyalkoxyalkyl)amino" as used herein refers to $R_{34}R_{35}N$-, wherein $R_{34}$ and $R_{35}$ are polyalkoxy residues appended to loweralkyl residues.

The term "((polyalkoxy)alkyl)(alkyl)amino" as used herein refers to $R_{36}R_{37}N$-, wherein $R_{36}$ is a polyalkoxy residue appended to a loweralkyl radical and $R_{37}$ is a loweralkyl residue.

The term "((heterocyclic)alkyl)(alkyl)amino" as used herein refers to $-NR_{71}R_{72}$ wherein $R_{71}$ is a heterocyclicalkyl group and $R_{72}$ is a loweralkyl group.

The term "((heterocyclic)alkyl)amino" as used herein refers to $-NHR_{73}$ wherein $R_{73}$ is a heterocyclic alkyl group.

The term "(heterocyclic)(alkyl)amino" as used herein refers to $-NR_{74}R_{75}$ wherein $R_{74}$ is a substituted or unsubstituted heterocyclic group and $R_{75}$ is a loweralkyl group.

The term "(alkylaminoalkyl)(alkyl)amino" as used herein refers to $-NR_{76}R_{77}$ wherein $R_{76}$ is an alkylaminoalkyl group and $R_{77}$ is a loweralkyl group.

The term "(dialkylaminoalkyl)(alkyl)amino" as used herein refers to $-NR_{78}R_{79}$ wherein $R_{78}$ is a dialkylaminoalkyl group and $R_{79}$ is a loweralkyl group.

The term "((alkoxy)(alkyl)aminoalkyl)(alkyl)amino" as used herein refers to $-NR_{80}R_{81}$ wherein $R_{80}$ is $-NR_{82}R_{83}$ appended to a loweralkyl radical wherein $R_{82}$ is an alkoxy group and $R_{83}$ is a loweralkyl group and $R_{81}$ is a loweralkyl group.

The term "((alkoxy)aminoalkyl)(alkyl)amino" as used herein refers to $-NR_{84}R_{85}$ wherein $R_{84}$ is $-NHR_{86}$ appended to a loweralkyl group and wherein $R_{86}$ is an alkoxy group and $R_{85}$ is a loweralkyl group.

The term "carboxyalkoxyalkyl" as used herein refers to a carboxy (-COOH) group appended to an

alkoxy group which is in turn appended to a loweralkyl radical.

The term "(alkoxycarbonyl)alkoxyalkyl" as used herein refers to an alkoxycarbonyl group (-C(O)O$R_{200}$ wherein $R_{200}$ is loweralkyl) appended to an alkoxy group which in turn is appended to a loweralkyl radical.

The term "carboxyalkylamino" as used herein refers to a carboxy group appended to the alkyl portion of an alkylamino radical.

The term "alkoxycarbonylalkylamino" as used herein refers to an alkoxycarbonyl group appended to the alkyl portion of an alkylamino radical.

The term "(amino)carboxyalkyl" as used herein refers to a loweralkyl radical to which is appended an amino group and a carboxy group.

The term "(amino)carboxyalkylamino" as used herein refers to an alkylamino radical, the alkyl portion of which has appended an amino group and a carboxy group.

The term "((N-protected)amino)carboxyalkyl" as used herein refers to a loweralkyl radical to which is appended an N-protected amino group and a carboxy group.

The term "((N-protected)amino)carboxyalkylamino" as used herein refers to an amino radical to which is appended an ((N-protected)amino)carboxyalkyl group.

The term "(alkylamino)carboxyalkyl" as used herein refers to a loweralkyl radical to which is appended an alkylamino group and a carboxy group.

The term "(alkylamino)carboxyalkylamino" as used herein refers to an amino radical to which is appended an (alkylamino)carboxyalkyl group.

The term "((N-protected)alkylamino)carboxyalkyl" as used herein refers to a loweralkyl radical to which is appended an (N-protected)alkylamino group and a carboxy group.

The term "((N-protected)alkylamino)carboxyalkylamino" as used herein refers to an amino radical to which is appended an ((N-protected)alkylamino)carboxyalkyl group.

The term "(dialkylamino)carboxyalkyl" as used herein refers to a loweralkyl radical to which is appended a dialkylamino group and a carboxy group.

The term "(dialkylamino)carboxyalkylamino" as used herein refers to an amino radical to which is appended an (dialkylamino)carboxyalkyl group.

The term "(amino)alkoxycarbonylalkyl" as used herein refers to a loweralkyl radical to which is appended an amino group and an alkoxycarbonyl group.

The term "(amino)alkoxycarbonylalkylamino" as used herein refers to an amino radical to which is appended an (amino)alkoxycarbonylalkyl group.

The term "(N-protected)amino)alkoxycarbonylalkyl" as used herein refers to a loweralkyl radical to which is appended an (N-protected)amino group and an alkoxycarbonyl group.

The term "(N-protected)amino)alkoxycarbonylalkylamino" as used herein refers to an amino radical to which is appended an (N-protected)amino)alkoxycarbonylalkyl group.

The term "(alkylamino)alkoxycarbonylalkyl" as used herein refers to a loweralkyl radical to which is appended an alkylamino group and an alkoxycarbonyl group.

The term "(alkylamino)alkoxycarbonylalkylamino" as used herein refers to an amino radical to which is appended an (alkylamino)alkoxycarbonylalkyl group.

The term "((N-protected)alkylamino)alkoxycarbonylalkyl" as used herein refers to a loweralkyl radical to which is appended an (N-protected)alkylamino group and an alkoxycarbonyl group.

The term "((N-protected)alkylamino)alkoxycarbonylalkylamino" as used herein refers to an amino radical to which is appended an ((N-protected)alkylamino)alkoxycarbonylalkyl group.

The term "(dialkylamino)alkoxycarbonylalkyl" as used herein refers to a loweralkyl radical to which is appended a dialkylamino group and an alkoxycarbonyl group.

The term "(dialkylamino)alkoxycarbonylalkylamino" as used herein refers to an amino radical to which is appended a (dialkylamino)alkoxycarbonylalkyl group.

The term "loweralkylcarbonyl" as used herein refers to $R_{87}$C(O)- wherein $R_{87}$ is a loweralkyl group, including, but not limited to acetyl, propionyl and the like.

The terms "alkoxy" and "thioalkoxy" as used herein refer to $R_{38}$O- and $R_{38}$S-, respectively, wherein $R_{38}$ is a loweralkyl group.

The term "alkenyloxy" as used herein refers to $R_{39}$O-, wherein $R_{39}$ is an alkyl group of 1 to 7 carbon atoms which contains at least one double bond.

The term "hydroxyalkoxy" as used herein refers to -OH appended to an alkoxy radical.

The term "dihydroxyalkoxy" as used herein refers to an alkoxy radical which is disubstituted with -OH radicals.

The term "arylalkoxy" as used herein refers to an aryl group appended to an alkoxy radical, including, but not limited to benzyloxy and the like.

The term "carboxy" as used herein refers to -COOH.

The term "alkoxycarbonyl" as used herein refers to $R_{102}C(O)$- wherein $R_{102}$ is an alkoxy group.

The term "polyalkoxy" as used herein refers to $R_{40}O$-, wherein $R_{40}$ is a straight or branched chain containing 1-5, $C_n$-O-$C_{n'}$ linkages wherein n and n′ are independently 1-3.

The terms "substituted carbonyloxy and substituted carbonyloxy analog" as used herein include $R_{10}$-Q-B-
wherein

B is
(i) NH,
(ii) -N(alkyl)-,
(iii) S,
(iv) O,
(v) $-CH_2-$,
(vi) $-NHCH_2-$ or
(vii) $-CH(OR_{52})-$ wherein $R_{52}$ is hydrogen, loweralkyl or loweralkylcarbonyl,

Q is
(i) -C(O)-,
(ii) -S(O)-,.
(iii) $-S(O)_2-$ or
(iv) $-CH(OR_{100})-$ wherein $R_{100}$ is hydrogen, loweralkyl or $-C(O)R_{101}$ wherein $R_{101}$ is loweralkyl and

$R_{10}$ is
(i) hydrogen,
(ii) loweralkyl,
(iii) haloalkyl,
(iv) cycloalkyl,
(v) aryl,
(vi) arylalkyl,
(vii) alkoxy,
(viii) alkenyloxy,
(ix) hydroxyalkoxy,
(x) dihydroxyalkoxy,
(xi) arylalkoxy,
(xii) arylalkoxyalkyl,
(xiii) thioalkoxyalkyl,
(xiv) loweralkylsulfinylalkyl,
(xv) loweralkylsulfonylalkyl,
(xvi) arylthioalkyl,
(xvii) arylsulfonylalkyl,
(xviii) amino,
(xix) alkylamino,
(xx) dialkylamino,
(xxi) (hydroxyalkyl)(alkyl)amino,
(xxii) (dihydroxyalkyl)(alkyl)amino,
(xxiii) aminoalkyl,
(xxiv) alkoxycarbonylalkyl,
(xxv) carboxyalkyl,
(xxvi) (N-protected)aminoalkyl,
(xxvii) (alkyl)aminoalkyl,
(xxviii) (N-protected)(alkyl)aminoalkyl,
(xxix) (dialkyl)aminoalkyl,
(xxx) (heterocyclic)alkyl,
(xxxi) heterocyclic,
(xxxii) aminocycloalkyl,
(xxxiii) aminoalkylamino,
(xxxiv) (aminoalkyl)(alkyl)amino,
(xxxv) arylalkylamino,
(xxxvi) (arylalkyl)(alkyl)amino,
(xxxvii) (alkoxyalkyl)(alkyl)amino,

(xxxvii) ((polyalkoxy)alkyl)(alkyl)amino,
(xxxviii) di-(alkoxyalkyl)amino,
(xxxix) di-(hydroxyalkyl)amino,
(xl) di-((polyalkoxy)alkyl)amino,
(xli) ((heterocyclic)alkyl)(alkyl)amino,
(xlii) ((heterocyclic)alkyl)amino,
(xliii) (heterocyclic)(alkyl)amino,
(xliv) (alkylaminoalkyl)(alkyl)amino,
(xlv) (dialkylaminoalkyl)(alkyl)amino,
(xlvi) ((alkoxy)(alkyl)aminoalkyl)(alkyl)amino,
(xlvii) ((alkoxy)aminoalkyl)(alkyl)amino,
(xlviii) polyalkoxy,
(xlix) (polyalkoxy)alkyl,
(l) carboxyalkoxyalkyl,
(li) (alkoxycarbonyl)alkoxyalkyl),
(lii) carboxyalkylamino,
(liii) alkoxycarbonylalkylamino,
(liv) (amino)carboxyalkyl,
(lv) (amino)carboxyalkylamino,
(lvi) ((N-protected)amino)carboxyalkyl,
(lvii) ((N-protected)amino)carboxyalkylamino,
(lviii) (alkylamino)carboxyalkyl,
(lix) (alkylamino)carboxyalkylamino,
(lx) (((N-protected)alkylamino)carboxyalkyl,
(lxi) ((N-protected)alkylamino)carboxyalkylamino,
(lxii) (dialkylamino)carboxyalkyl,
(lxiii) (dialkylamino)carboxyalkylamino,
(lxiv) (amino)alkoxycarbonylalkyl,
(lxv) (amino)alkoxycarbonylalkylamino,
(lxvi) ((N-protected)amino)alkoxycarbonylalkyl,
(lxvii) ((N-protected)amino)alkoxycarbonylalkylamino,
(lxviii) (alkylamino)alkoxycarbonylalkyl,
(lxix) (alkylamino)alkoxycarbonylalkyl,
(lxx) (alkylamino)alkoxycarbonylalkylamino,
(lxxi) (((N-protected)alkylamino)alkoxycarbonylalkyl,
(lxxii) ((N-protected)alkylamino)alkoxycarbonylalkylamino,
(lxxiii) (dialkylamino)alkoxycarbonylalkyl or
(lxxiv) ((dialkylamino)alkoxycarbonylalkylamino.

The term "halo" as used herein refers to Cl, Br, F or I substituents.

The term "halobenzyl" as used herein refers to a halo substituent appended to the phenyl ring of a benzyl radical.

The term "halophenyl" as used herein refers to a halo substituent appended to a phenyl radical.

The term "aryl" as used herein refers to a monocyclic or bicyclic carbocyclic ring system having one or more aromatic rings including, but not limited to, phenyl, naphthyl, tetrahydronaphthyl, indanyl and the like. Aryl groups can be unsubstituted or substituted with one, two or three substituents independently selected from loweralkyl, haloalkyl, alkoxy, thioalkoxy, amino, alkylamino, dialkylamino, hydroxy, halo, mercapto, nitro, carboxaldehyde, carboxy, alkoxycarbonyl and carboxamide.

The term "heterocyclic group" or "heterocyclic" as used herein refers to any 3-, 4-, 5- or 6-membered ring containing a heteroatom selected from oxygen, nitrogen and sulfur, or a 5- or 6-membered ring containing two or three nitrogen atoms; or one nitrogen and one oxygen atom; or one nitrogen and one sulfur atom; wherein the 5-membered ring has 0-2 double bonds and the 6-membered ring has 0-3 double bonds; wherein the nitrogen and sulfur heteroatoms may optionally be oxidized; wherein the nitrogen heteroatom may optionally be quaternized; and including any bicyclic group in which any of the above heterocyclic rings is fused to a benzene ring or another 5- or 6-membered heterocyclic ring independently as defined above. Heterocyclics in which nitrogen is the heteroatom are preferred. Preferred heterocyclics include: pyrryl, pyrrolinyl, pyrrolidinyl, pyrazolyl, pyrazolinyl, pyrazolidinyl, imidazolyl, imidazolinyl, imidazolidinyl, pyridyl, piperidinyl, pyrazinyl, piperazinyl, N-methyl piperazinyl, azetidinyl, N-methyl azetidinyl, pyrimidinyl, pyridazinyl, oxazolyl, oxazolidinyl, isoxazolyl, isoxazolidinyl, morpholinyl, thiazolyl, thiazolidinyl,

isothiazolyl, isothiazolidinyl, indolyl, quinolinyl, isoquinolinyl, benzimidazolyl, benzothiazolyl, benzoxazolyl, furyl, thienyl, triazolyl and benzothienyl.

Heterocyclics can be unsubstituted or monosubstituted or disubstituted with substitutents independently selected from hydroxy, halo, oxo (=O), alkylimino ($R^*N=$ wherein $R^*$ is a loweralkyl group), amino, alkylamino, dialkylamino, alkoxy, thioalkoxy, polyalkoxy, loweralkyl, cycloalkyl or haloalkyl.

The most preferred heterocyclics include imidazolyl, pyridyl, piperazinyl, N-methyl piperazinyl, azetidinyl, N-methyl azetidinyl, thiazolyl, thienyl, triazolyl and the following:

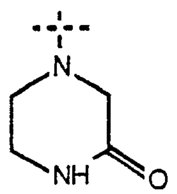 , 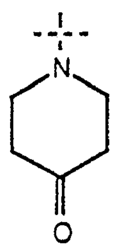 , 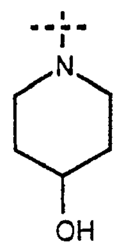 , 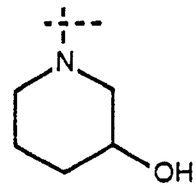 ,

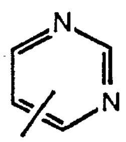 , 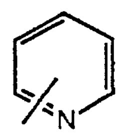 , 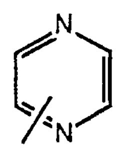 , 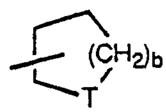 or 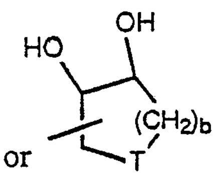

wherein b is 1 or 2 and T is N, NH, O, S, provided that T is the point of connection only when T is N,

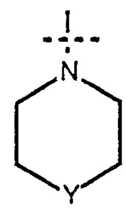

wherein Y is NH, N-loweralkyl, O, S, or $SO_2$, or

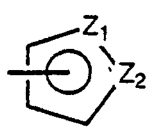 , 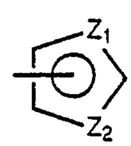 , 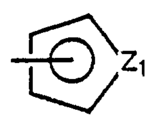

(i) (ii) (iii)

wherein the symbols (i), (ii) and (iii) represent 5-membered heterocycles containing one or more heteroatoms and containing 2 double bonds; wherein $Z_1$ is N, O, or S and not the point of connection and $Z_2$ is N when it is the point of connection or NH, O or S when it is not the point of connection.

The term "N-protecting group" or "N-protected" as used herein refers to those groups intended to protect the N-terminus of an amino acid or peptide or to protect an amino group against undesirable reactions during synthetic procedures or to prevent the attack of exopeptidases on the compounds or to increase the solubility of the compounds and includes but is not limited to sulfonyl, acyl, acetyl, pivaloyl, t-butyloxycarbonyl (Boc), carbonylbenzyloxy (Cbz), benzoyl or an L- or D-aminoacyl residue, which may itself be N-protected similarly.

The term "O-protecting group" as used herein refers to a substituent which protects hydroxyl groups against undesirable reactions during synthetic procedures and includes but is not limited to substituted methyl ethers, for example methoxymethyl, benzylozymethyl, 2-methoxyethoxy-methyl, 2-(trimethylsilyl)-ethoxymethyl, benzyl and triphenylmethyl; tetrahydropyranyl ethers; substituted ethyl ethers, for example, 2,2,2-trichloroethyl and t-butyl; silyl ethers, for example, trimethylsilyl, t-butyldimethylsilyl and t-butyldiphenylsilyl; cyclic acetals and ketals, for example, methylene acetal, acetonide and benzylidene acetal; cyclic ortho esters, for example, methoxymethylene; cyclic carbonates; and cyclic boronates.

The terms "Ala", "His", "Leu", "Phe", "Tyr", "Cys", "Gly", "Lys", "Sar", and "Pro" as used herein refer to alanine, histidine, leucine, phenylalanine, tyrosine, cysteine, glycine, lysine, sarcosine and proline, respectively. In general, the amino acid abbreviations follow the IUPAC-IUB Joint Commission on Biochemical Nomenclature for amino acids and peptides (Eur. J. Biochem. 1984, 158 , 9-31).

The compounds of the invention may be made as shown in Schemes 1 and 2. The process shown in Scheme 1 shows the preparation of the key intermediate VI, which upon reduction of the ketone function and opening of the oxazolidinone ring under basic hydrolysis provides the important intermediates difluorodiols VIIIa and VIIIb which when coupled to peptidyl fragments at the N-terminal provided the renin-inhibiting compounds.

Scheme 2 shows the coupling of intermediates IX and X to provide the compounds of the invention (I). This coupling can be accomplished using standard peptide coupling methods. Activated forms of the carboxylic acid X can be used in the coupling reaction. These activated forms include acid halides and activated esters which include formic and acetic acid derived anhydrides, anhydrides derived form alkoxycarbonyl halides such as isobutyloxycarbonylchloride, N-hydroxysuccinimide derived esters, N-hydroxyphthalimide derived esters, N-hydroxybenzotriazole derived esters, N-hydroxy-5-norbornene-2,3-dicarboxamide derived esters, 4-nitrophenol derived esters, 2,4,5-trichlorophenol derived esters and the like.

SCHEME 1

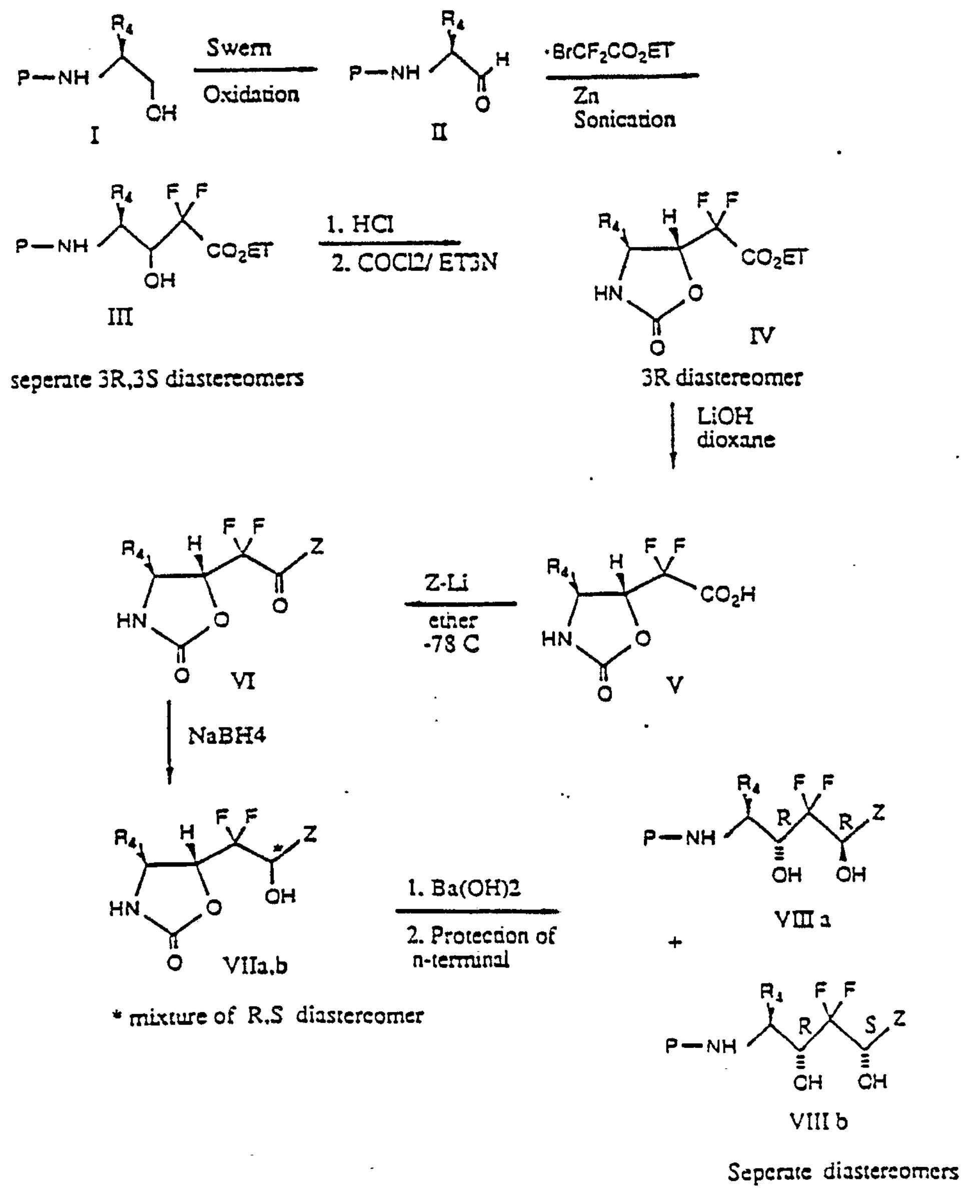
Swern
Oxidation
·BrCF2CO2ET
Zn
Sonication
1. HCl
2. COCl2/ ET3N
seperate 3R,3S diastereomers
3R diastereomer
LiOH
dioxane
Z-Li
ether
-78 C
NaBH4
1. Ba(OH)2
2. Protection of
n-terminal
* mixture of R,S diastereomer
+
Seperate diastereomers

Scheme 2

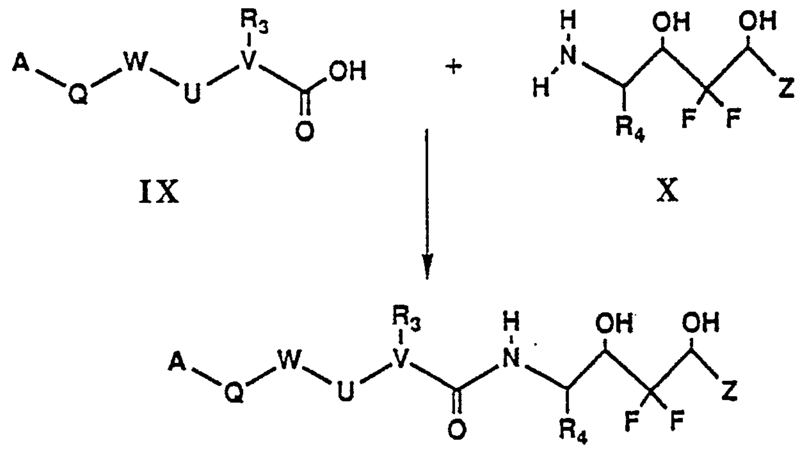

IX

X

I

Intermediates useful for the preparation of the novel compounds of this invention include compounds of the formula:

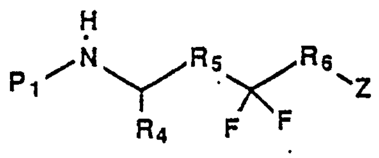

$P_1$ is hydrogen or an N-protecting group.

$R_4$ is

(1) loweralkyl,

(2) cycloalkylmethyl or

(3) benzyl.

$R_5$ is

(1) -CH(OH)- or

(2) -C(O)-.

$R_6$ is

(1) -CH(OH)- or

(2) -C(O)-.

Z is

(1) lower alkyl,

(2) aryl,

(3) arylalkyl,

(4) cycloalkyl,

(5) cycloalkylalkyl,

(6) heterocyclic or

(7) (heterocyclic)alkyl.

Other intermediates useful for the preparation of the novel compounds of this invention include compounds of the formula:

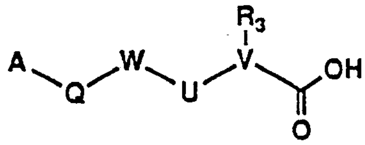

or an acid halide or activated ester derivative thereof.

A is

(1) hydrogen,
(2) loweralkyl,
(3) functionalized alkyl,
(4) aryl,
(5) heterocyclic,
(6) $-OR_7$ or $-SR_7$ wherein $R_7$ is
  (i) hydrogen,
  (ii) loweralkyl,
  (iii) functionalized alkyl,
  (iv) aryl,
  (v) heterocyclic,
  (vi) aryl-C(O)- or
  (vii) heterocyclic-C(O)-,
(7) carboxy,
(8) alkoxycarbonyl,
(9) functionalized amino,
(10) functionalized hydroxyalkyl or
(11) substituted carbonyloxy or substituted carbonyloxy analog.

W is

(1) -C(O)-,
(2) -CH(OH)- or
(3) $-N(R_2)-$ wherein $R_2$ is hydrogen or loweralkyl.

U is

(1) -C(O)-,
(2) $-CH_2-$ or
(3) $-N(R_2)-$ wherein $R_2$ is hydrogen or lower alkyl, with the proviso that when W is -CH(OH)-then U is $-CH_2-$ and with the proviso that U is -C(O)- or $-CH_2-$ when W is $-N(R_2)-$.

V is

(1) -CH-,
(2) -C(OH)- or
(3) -C(halogen)- with the proviso that V is -CH-when U is $-N(R_2)-$.

Q is $-CH(R_1)-$ or $-C(=CHR_{1a})-$ wherein $R_1$ is

(1) loweralkyl,
(2) cycloalkylalkyl,
(3) arylalkyl,
(4) (heterccyclic)alkyl,
(5) 1-benzyloxyethyl,
(6) phenoxy,
(7) thiophenoxy or
(8) anilino, provided that B is $-CH_2-$ or -CH(OH)-or A is hydrogen when $R_1$ is phenoxy, thiophenoxy or anilino and $R_{1a}$ is aryl or heterocyclic.

$R_3$ is

(1) loweralkyl,
(2) loweralkenyl,
(3) hydroxyalkyl,
(4) alkoxyalkyl,
(5) ((alkoxy)alkoxy)alkyl,
(6) Carboxyalkyl,
(7) (thioalkoxy)alkyl,

(8) azidoalkyl,
(9) aminoalkyl,
(10) (alkyl)aminoalkyl,
(11) (dialkyl)aminoalkyl,
(12) (alkoxy)(alkyl)aminoalkyl,
(13) (alkoxy)aminoalkyl,
(14) benzyl or
(15) (heterocyclic)methyl.

Acid halide derivatives of the above intermediates include the acid chloride. Activated ester derivatives of the above intermediates include activated esters commonly used by those skilled in the art for activating carboxylic acid groups for coupling with an amine to form a peptide bond, including, but not limited to formic and acetic acid derived anhydrides, anhydrides derived from alkoxycarbonyl halides such as isobutyloxycarbonylchloride and the like, N-hydroxysuccinimide derived esters, N-hydroxyphthalimide derived esters, N-hydroxybenzotriazole derived esters, N-hydroxy-5-norbornene-2,3-dicarboxamide derived esters, 4-nitrophenol derived esters, 2,4,5-trichlorophenol derived esters and the like.

The following Examples will serve to further illustrate preparation of the novel compounds of the present invention.

## Example 1

Ethyl 4(S)-((t-butyloxycarbonyl)amino)-5-cyclohexyl-2,2-difluoro-3(R,S)-hydroxypentanoate.

To a suspension of 1.2 g (17 mmole) of activated zinc in 5 ml of tetrahydrofuran under argon in a sonicating bath was added slowly a solution of 1.7 g (6.8 mmole) of Boc-L-cyclohexylalaninal and 2.34 ml (18.4 mmole) of ethyl bromodifluoroacetate in 30 ml of tetrahydrofuran. After complete addition, the solution was sonicated for an additional 30 min. The mixture was then added to 1 M $KHSO_4$ and extracted with dichloromethane (3 X 100 ml), dried with $Na_2SO_4$, filtered and concentrated in vacuo. The residual oil was purified by silica gel column chromatography (15-30% ethyl acetate in hexane) to give 1.22 g (75%) of two diasteromers.

3(R) diastereomer: $^1$H NMR ($CDCl_3$) $\delta$ 1.37 (t,3H, J=7.0 Hz), 1.46 (S,9H), 4.35 (q,2H, J=7.0 Hz). m.p. 73-74.5 °C.

Anal. ($C_{18}H_{31}NO_5F_2$)C,H,N.

3(S) diastereomer: $^1$H NMR ($CDCl_3$) $\delta$ 1.37 (t,3H, J=7.5 Hz), 1.45 (S,9H), 4.31 (1,2H, J=7.5 Hz); m.p. 115 °-117 °C.

Anal. ($C_{18}H_{31}NO_5F_2$)C,H,N.

## Example 2

2-Oxazolidinone derivative of Ethyl 4(S)-amino-5-cyclohexyl-2,2-difluoro-3(R)-hydroxypentanoate.

To 50 mg of the 3(R) isomer from Example 1 was added 1 ml of 4 M HCl in dioxane. The solution was stirred at RT for 30 min. The concentrated residue was dissolved in dichloromethane and treated with 0.1 ml of triethylamine and excess phosgene in toluene (10% solution). After stirring at RT for 1 hr, the crude product was purified by silica gel column chromatography (10% ethyl acetate in hexane) to give 32 mg of desired product. $^1$H NMR ($CDCl_3$) $\delta$ 1.38 (t,3H, J=7 Hz), 4.08 (m,1H), 4.38 (q, 2H, J=7 Hz), 4.58 (ddd,1H, J=4.5,6.0,15 Hz), 6.05 (br S,1H).

Anal. ($C_{14}H_{21}NO_4F_2$)C,H,N.

## Example 3

2-Oxazolidinone derivative of Ethyl 4(S)-amino-5-cyclohexyl-2,2-difluoro-3-(S)hydroxypentanoate.

Using the same procedure as in Example 2, and using the 3(S) isomer from Example 1 as the starting material provided the desired product $^1H$ NMR ($CDCl_3$) δ 1.38 (t,3H,J=7.5 Hz), 4.28 (ddd, 1H, J=3,7.5,12 Hz), 4.38 (q,2H, J=7.5 Hz), 5.02 (ddd,1H,J=6,9,19.5 Hz), 5.63 (br S,1H).
Anal. ($C_{14}H_{21}NO_4F_2$)C,H,N.

Example 4

4(S)-cyclohexylmethyl-5(R)-(4′(4′,4′-difluoro-3′-oxo-2′-methyl-buthyl))-2-oxazolidinone.

The hydrolysis of 2.5 g of the product in Example 3 by lithium hydroxide in aqueous methanol provided 2.3 g of the corresponding carboxylic acid. The acid was dissolved in 40 ml of THF and cooled to -78° C. To the vigorously stirred solution was added 18 ml of isopropyl lithium solution in pentane (12.4% by wt.). After 30 min, the solution was slowly warmed to 0° C and stirred for an additional 30 min. The reaction was carefully quenched with water and extracted with ethyl acetate (3 X 100 ml), dried and concentrated in vacuo. The crude product was purified by silica gel column chromatography (20% ethyl acetate in hexane) to give 1.36 g of desired product. $^1H$ NMR ($CDCl_3$) δ 1.20 (t,6H, J=6.3 Hz), 3.17 (d of heptet, 1H, J=1.8,6.6 Hz), 4.06 (m,1H), 4.62 (ddd,1H, J=4.5,6.0,20.4 Hz), 5.63 (br S,1H). Anal. ($C_{14}H_{23}NO_3F_2$)C,H,N.

Example 5

4(S)-cyclohexylmethyl-5(R)-(3′-(3′,3′-difluoro-2′-oxo-propyl))-2-oxazolidinone.

Using the same procedure as described in Example 4, but replacing isopropyl lithium with methyl lithium gave the desired product. $^1H$ NMR ($CDCl_3$), δ 2.42 (d,3H, J=3 Hz), 4.07 (m,1H), 4.56 (td,1H, J=4.5,16 Hz), 5.58 (br S,1H).

Example 6

4(S)-cyclohexylmethyl-5(R)(4′-4′,4′-difluoro-3(R,S)-hydroxy-2′-methylbutyl))-2-oxazolidinone.

To the product of Example 4 (1.0 g, 3.4 mmole) in 20 ml of methanol at 0° C was added 125 mg of sodium borohydride. After 10 min the reaction was quenched by addition of excess acetone. The solution was concentrated and the residual oil was dissolved in ethyl acetate and washed with brine. The aqueous layer was extracted with ethyl acetate (2 X 50 ml). The combined ethyl acetate solution was dried with $MgSO_4$ and concentrated to give a mixture of 3′(R) and 3′(S) diastereomers.
3′(R) isomer: $^1H$ NMR ($CDCl_3$): δ 1.04 (t,6H, J=7.5 Hz), 2.12 (m,1H), 3.82 (m,1H), 4.10 (m,1H), 4.63 (dd,1H, J=4.5,22.5 Hz), 5.38 (br S,1H).
Anal. ($C_{14}H_{25}NO_3F_2$)C,H,N.
3′(S) isomer: $^1H$ NMR ($CDCl_3$): δ 1.01 (d,3H, J=6Hz), 1.07 (d,3H, J= 2.10 (m,1H), 3.80 (m,1H), 4.15 (m,1H), 4,40 (td, 1H, J=6,15.5 Hz), 5.62 (br S,1H).

Anal. ($C_{14}H_{25}NO_3F_2$)C,H,N.

## Example 7

### 2(S)-Benzyloxycarbonylamino-1-cyclohexyl-4,4-difluoro-3(R),5(R)-dihydroxy-6-methylheptane.

To a solution of 0.9 g (3.0 mmole) of the 3′(R) isomer in Example 6 in 50 ml of dioxane and water was added 1.05 g (2.1 eq) of barium hydroxide hydrate. The reaction mixture was heated to reflux for 18 hr. and cooled to RT and filtered. The filtrate was concentrated in vacuo and the crude product was dissolved in 50 ml of dichloromethane and 1.0 g of N-(benzyloxycarbonyloxy)succinimide was added. After 1 hr. at RT the solution was washed with satd. $NaHCO_3$ and extracted with dichloromethane (3 X 50 ml), dried with $Na_2SO_4$ and filtered. The filtrate was concentrated in vacuo to give a pale yellow oil which was purified by silica gel column chromatography (10% EtOAc in $CH_2Cl_2$) to give 900 mg of the desired product. $^1H$ NMR ($CDCl_3$) $\delta$ 1.01 (d,3H, J=5Hz), 1.03 (d,3H, J=5Hz), 2.07 (m,1H), 2.42 (br d,1H), 3.70-3.95 (m,3H), 4.07 (m,1H), 5.02 (br d,1H), 5.11 (S,2H), 7.30-7.36 (m,5H).
Anal. ($C_{22}H_{33}NO_4F_2$)C,H,N.

## Example 8

### 2(S)-Benzyloxycarbonylamino-1-cyclohexyl-4,4-difluoro-3(R),5(S)-dihydroxy-6-methylheptane.

Using the same procedure as described in Example 7, except replacing the 3′(R) isomer in Example 6 with the 3′(S) isomer in Example 6 provided the desired product. $^1H$ NMR ($CDCl_3$) $\delta$ 0.98 (d,3H, J=6Hz), 1.03 (d,3H, J=6Hz), 2.10 (m,1H), 2.45 (br m, 1H), 3.50 (br S,1H), 3.74-3.96 (m,2H), 4.15 (m,1H), 5.08 (br d, 1H), 5.10 (s,2H), 7.30-7.38 (m,5H).
Anal. ($C_{22}H_{33}NO_4F_2$)C,H,N.

## Example 9

### 2(S)-Amino-1-cyclohexyl-4,4-difluoro-3(R),5(R)-dihydroxy-6-methylheptane.

A solution of 700 mg of the product from Example 7 in 10 ml of methanol was stirred vigorously under a hydrogen atmosphere using 10% Pd/C as catalyst. After 30 min, the catalyst was filtered off and the solution concentrated to give 470 mg the desired product. Mass spectrum: $M^+$ = 279.

## Example 10

### 2(S)-Amino-1-cyclohexyl-4,4-difluoro-3(R),5(S)-dihydroxy-6-methylheptane.

Using the same procedure as described in Example 9, but using the resultant product from Example 8 as starting material provided the desired product. Mass spectrum: $M^+$ = 279.

Example 11

4(S)-cyclohexylmethyl-5(R)-(3′-(3′,3′-difluoro-2′(R,S)-hydroxy-propyl))-2-oxazolidinone.

Using the same procedure as described in Example 6, but using the product from Example 5 as starting material provided the desired product as a 1:1 mixture of 2′(R) and 2′(S) diastereomers which were not separated. Mass spectrum: $M^+$ = 277.

Example 12

2(S)-Benzyloxycarbonylamino-1-cyclohexyl-4,4-difluoro-3(R),5(R,S)-dihydroxyhexane.

Using the procedure described in Example 7, but using the product from Example 11 as starting material provided the desired product. Mass spectrum: $M^+$ = 385.

Example 13

2(S)-Amino-1-cyclohexyl-4,4-difluoro-3(R),5(R,S)-dihydroxyhexane.

Using the procedure described in Example 9, but using the product from Example 12 provided the desired product.
Mass spectrum: $M^+$ = 251.

Example 14

Boc-His Amide of 2(S)-Amino-1-cyclohexyl-4,4-difluoro-3(R),5(R)-dihydroxy-6-methylheptane.

To a solution of 470 mg of the product from Example 9 in 20 ml of dimethylformamide (DMF) was added 734 mg of 1-hydroxybenzotriazole (HOBt) and 455 mg of Boc-His-OH. The mixture was cooled to -20° C and 0.3 ml of triethylamine (TEA) was added, followed by 410 mg of 1-(3-dimethylaminopropyl)-3-ethyl-carbodiimide (EDAC). The mixture was stirred at -20° C for 2 hr and then at RT for 16 hr. It was poured into satd. $NaHCO_3$ solution and extracted with ethyl acetate (3 X 50 ml). The ethyl acetate solution was washed with water and brine, then dried over $Na_2SO_4$ and concentrated. The residual solid was purified by silica gel column chromatography (5% MeOH in $CH_2Cl_2$) to provide 580 mg of the desired product as a white solid. $^1H$ NMR ($CDCl_2$). δ 1.02 (d,6H), 1.48 (S,9H), 6.88 (s,1H), 7.57 (s,1H).

Example 15

Boc-(4-Thiazolyl)alanine Amide of 2(S)-Amino-1-cyclohexyl-4,4-difluoro-3(R),5(R)-dihydroxy-6-methylheptane.

Using the procedure described in Example 14, but replacing Boc-His-OH with Boc-(4-thiazolyl)alanine provided the desired product.

Example 16

(E)-2-((4-Morpholinylcarbonyl)methyl) cinnamic acid.

To a stirred -12° C solution of α-ethyl α-benzalsuccinate (J. Am. Chem. Soc. 1968, 90 , pg. 3495) (1.6 g, 6.83 mmole) in anhydrous THF (50 ml) were added N-methylmorpholine (1 ml, 9.09 mmole) and isobutyl chloroformate (1.2 ml, 9.25 mmole) sequentially. After 5 min, a -12° C solution of morpholine (0.8 ml, 9.17 mmole) was added. After 1 hr, the solution was filtered and the filtrate concentrated. The residual oil was dissolved in 200 ml of EtOAc and washed successively with 10% sodium bisulfate, aqueous $NaHCO_3$ and brine. The organic layer was dried with $Na_2SO_4$, filtered and evaporated. The residue was chromatographed on silica gel with 2% MeOH in $CH_2Cl_2$ as eluent to give 985 mg (48%) of ethyl 2-((4-morpholinylcarbonyl)-methyl)-trans-cinnamate. $^1H$ NMR ($CDCl_3$) δ 1.35 (t,3H, J=7Hz), 3.50 (S,2H), 3.51-3.72 (m,SH), 4.27 (q,2H, J=7Hz), 7.35-7.40 (m,5H), 7.92 (s,1H).
Hydrolysis of the above ester using LiOH in dioxane/$H_2O$ gave the corresponding acid in 92% yield. $^1H$ NMR ($CDCl_3$): δ 3.ao (s,2H), 3.55-3.70 (m,8H), 7.32-7.40 (m,5H), 7.90 (s,1H).

Example 17

3-Benzyloxycarbonylamino-3-methylbutanoic Acid.

A solution of 2,2-dimethyl-3-carbomethyoxypropionic acid (LeMaul, Bull. Soc. Chim. Fr., 828 (1965)), (20 g, 0.125 mol), diphenylphosphorylazide (34.3 g, 0.125 mol) and triethylamine was heated in toluene (150 ml) at 100° C for 2 h. After cooling to 5° C, the toluene solution was washed successively with 0.5 M HCl, aqueous $NaHCO_3$ and brine. Evaporation of the dried solution gave a residue which was chromatographed on silica gel eluting with 60/40 hexane-ether. There was obtained 13 g of methyl 3-isocyanato-3-methyl-butanoate as a mobile liquid. A solution of this material in toluene (20 ml) was treated with benzyl alcohol (13 ml) and the resulting mixture heated at reflux for 40 h. Evaporation of the toluene left a residue which was dissolved in methanol (125 ml) and then treated with a solution of NaOH (6.6 g, 0.165 mmol) in 22 ml of water. After 5 h, the reaction mixture was partially evaporated, washed with ether and acidified with 6N HCl. Extraction with methylene chloride and evaporation gave 21 g of the desired product. NMR (300 MHz, $CDCl_3$): 1.42 (s,6H), 2.78 (s,2H), 5.08 (s,2H).

Example 18

Cbz-((β,β-di-Me)-β-Ala)-Phe-$OCH_3$.

A 4.0 g sample of 3-benzyloxycarbonylamino-3-methylbutanoic acid was coupled to phenylalanine methyl ester hydrochloride (3.43 g) using the mixed anhydride procedure. Purification of the crude product by flash chromatography eluting with 65/35 ether/hexane gave an 86% yield of product. NMR (300 MHz, $CDCl_3$): 1.32 (s,3H), 1.34 (s,3H), 2.46 (d,1H), 2.63 (d,1H), 2.98 (dd,1H), 3.09 (dd,1H), 3.70 (s,3H), 4.86 (dd,1H), 4.97 (d,1H), 5.2 (d,1H), 5.3 (s,1H), 6.13 (d,1H)

Example 19

Cbz(β,β-di-Me)-β-Ala)-Phe-OH.

To a 0° C solution of Cbz-(β,β-di-Me)-β-Ala)-Phe-$OCH_3$ (1.5 g, 3.63 mmol) in dioxane (15 ml) was added a solution of lithium hydroxide (0.174 g, 4.15 mmol) in water (7.5 ml). After stirring for 1 hr at 0-5° C, the reaction mixture was diluted with cold water and extracted two times with ether. The aqueous portion was acidified with 6N HCl and extracted with ether. The organic extract was washed with brine and evaporated to give an 87% yield of product as a viscous liquid.

Example 20

Cbz-((β,β-di-Me)-β-Ala)-(Me)Tyr-$OCH_3$.

Using the procedure of Example 18 and replacing phenylalanine methyl ester hydrochloride with O-methyltyrosine methyl ester hydrochloride provided the desired material.

Example 21

Cbz-(β,β-di-Me)-β-Ala)-(Me)Tyr-OH.

Using the procedure of Example 19 with the resultant compound from Example 20 gave the desired material.

Example 22

2(S)-(((4-Morpholinyl)carbonyl)oxy)-3-phenylpropionic Acid Methyl Ester.

To L-phenyllactic acid methyl ester (3.2 g) was added 150 ml of 12.5% phosgene in toluene and 25 drops of dimethylformamide. After stirring for 16 h at room temperature, the solvent was evaporated and the residue chased several times with benzene. The resulting product was dissolved in methylene chloride (50 ml), cooled to 0° C and treated by dropwise addition with 3.86 g (0.044 mmol) of morpholine. The reaction mixture was stirred for 2 h at 0-5° C and then distributed between 0.5 N HCl and methylene chloride. The organic phase was washed with aqueous $NaHCO_3$ and brine and evaporated to a residue. Flash chromatography on silica gel eluting with 2:1 ether/hexane gave a 65% yield of product. NMR (300 MHz): 3.08 (dd,1H), 3.20 (dd,1H), 3.8 (s,3H), 5.19 (dd,1H).

Example 23

2(S)-((4-Morpholinyl)carbonyl)oxy-3-phenylpropionic Acid.

Using the hydrolysis procedure of Example 19, the title compound was obtained in 90% yield.

Example 24

(4R)-3-(3-Phenylpropionyl)-4-(2-propyl)-oxazolidine-2-one.

To a stirred solution of 4-(2-propyl)-oxazolidine-2-one in anhydrous tetrahydrofuran (250 ml) under a nitrogen atmosphere at -78 °C were added in a dropwise fashion a solution of n -butyllithium in hexane (50 ml, 77.4 mmol) over 5 to 10 min. After stirring an additional 20 min at -78 °C 3-phenylpropionyl chloride (12.7 ml, 85.2 mmol) was added neat. The reaction was warmed to room temperature and stirred 1 to 2 h. The reaction was quenched by adding 100 ml of saturated aqueous ammonium chloride and the volatiles removed by rotary evaporation. The resulting aqueous residue was extracted three times with ether and the combined organic phases were washed with brine, dried ($Na_2SO_4$), filtered and concentrated in vacuo . Recrystallization from hexanes/ethyl acetate provided the title compound (16.6 g, 82%). m.p. = 86.5 to 87.5 °C.
Mass spectrum: $(M+NH_4)^+$ = 279, $(M+H)^+$ = 262.

Example 25

(4R)-3((2R)-3-t-butyloxycarbonyl-2-benzylpropionyl)-4-(2-propyl)-oxazolidine-2-one.

To a stirred solution of the product resulting from Example 24 (2.28 g, 8.72 mmol), in anhydrous tetrahydrofuran (30 ml) under a nitrogen atmosphere at -78 °C was added a solution of sodium hexamethyldisilylamide (9.6 ml, 9.59 mmol) in tetrahydrofuran. After stirring for 30 min at -78 °C, t-butyl bromoacetate (2.21 g, 11.34 mmol) was added in anhydrous tetrahydrofuran and the resulting solution stirred 1 h at -78 °C. The reaction was quenched by adding 20 ml of saturated aqueous ammonium chloride and partitioned between water and ether. The aqueous layer was drawn off and extracted with ether. The combined organic phases were washed with 10% aqueous HCl, saturated aqueous $NaHCO_3$, and brine, dried ($Na_2SO_4$), filtered, and concentrated in vacuo . Recrystallization from acetone/hexanes provided the desired purified product (2.59 g, 79%). m.p. = 167-168 °C.
Mass spectrum: $(M+NH_4)^+$ = 393, $(M+H)^+$ = 376.

Example 26

Benzyl-(2R)-3-t-butyloxycarbonyl-2-benzylpropionate.

To a stirred solution of dry benzyl alcohol (0.55 ml, 5.33 mmol) in anhydrous tetrahydrofuran (18 ml) under a nitrogen atmosphere at 0 °C was added a hexane solution of N-butyllithium (2.58 ml; 4.00 mmol). To this solution was added the product from Example 25 in anhydrous tetrahydrofuran (10 ml). After stirring 1 h at 0 °C the reaction was quenched by adding excess saturated aqueous ammonium chloride. The volatiles were removed by rotary evaporation and the resulting aqueous residue extracted two times with ether. The combined organic layers were washed with brine, dried ($Na_2SO_4$), filtered, and concentrated in vacuo provided an oil which was purified by chromatography on $SiO_2$ (15% ethyl acetate/hexanes) to provide the desired product (0.89 g, 94%) as a colorless oil. Mass spectrum: $(M)^+$ = 354.

Example 27

Benzyl-(2R)-3-carboxy-2-benzylpropionate

The product from Example 26 (0.52 g, 1.47 mmol) was dissolved in a 1:1 (v:v) solution (6 ml) of trifluoroacetic acid and dichloromethane and stirred at room temperature for 1 h. The volatiles were removed in vacuo to provide the title compound (0.437 g, 100%) as an oil which crystallized on standing. The unpurified material was of sufficient purity to employ in subsequent steps. Mass spectrum: $(M)^+$ =298.

Example 28

Benzyl-(2R)-2-benzyl-3-(N-morpholinocarbamoyl)-propionate.

The product from Example 27 (0.438 g, 1.47 mmol), diphenylphosphoryl azide (317 ml, 1.47 mmol), and triethylamine (205 ml, 1.47 mmol), in dry benzene (6 ml) were refluxed for 3 to 5 h to provide a solution of the derived isocyanate which was cooled to 0° C and treated with morpholine (141 µl, 1.62 ml). The cooling bath was removed and the reaction stirred for 1 h. The reaction mixture was poured into 10% aqueous HCl and extracted two times with ether. The combined organic layers were washed successively with saturated aqueous $NaHCO_3$ and brine, dried ($Na_2SO_4$), filtered and concentrated in vacuo to provide the unpurified product. The desired product (0.403 g, 72%) was obtained in pure form after chromatography on $SiO_2$ (3% methanol/chloroform) as a thick oil which formed an amorphous solid on standing. Mass spectrum: $(M)^+$ = 382. NMR (300 MHz, $CDCl_3$, ppm, TMS as internal standard) 7.12-7.40 (m,10H), 5.18 (AB; J=12.6 Hz; 2H), 4.8 (d,d,; J=5.7 Hz; 1H), 3.59 (d,d,d; J=5.4, 8.4, 14,4 Hz; 1H), 3.13 (d,d; J=6.0, 6.0 Hz; 4H), 2.8-3.10 (m, 3H).

Example 29

Benzyl-(2R)-2-benzyl-3-(ethoxycarbamoyl)-propionate.

The procedure as described in Example 28 was followed except absolute ethanol was employed in lieu of morpholine. Mass spectrum: $(M)^+$ = 341. NMR (300 MHz, $CDCl_3$, ppm, TMS as internal standard) 7.1-7.4 (m,10H), 5.17 (S,,2H) 4.96 (br s, 1H) ), 4.07 (d,d,d; J=6.6, 6.6, 6.6 Hz 2H), 3.25-3.5 (2 br ABX,2H), 2.9-3.05 (m,2H), 2.75-2.88 (br m,1H), 1.23 (d,d; J=6.6, 6.6 Hz; 3H).

Example 30

(2R)-2-Benzyl-3-(morpholinocarbamoyl)-propionic Acid.

The product from Example 28 (0.315 g, 0.86 mmol) was dissolved in ethyl acetate (5 ml) and syringed into a flask charged with 10% Pd/C (_0.3 g). The resulting suspension was exposed to 1 atm of gaseous hydrogen for 2 to 4 h. The catalyst was removed by filtration through a celite pad. The filtrate was concentrated in vacuo to provide the desired compound (0.21 g, 88%) as a cream colored foam which was employed without further purification. Mass spectrum: $(M+H)^+$ = 278.

Example 31

(2R)-2-Benzyl-3-ethoxycarbamoylpropionic Acid.

The procedure as described in Example 30 was followed employing the product from Example 29 in lieu of that from Example 28. Mass spectrum: $(M+H)^+ = 252$.

Example 32

Benzyl (2R)-2-Benzyl-3-morpholinocarbonylpropionate.

The product of Example 27 and morpholine were converted to the title compound using the mixed anhydride method of coupling. Mass spectrum: $(M)^+ = 367$.

Example 33

(2R)-2-Benzyl-3-morpholinocarbonylpropionic Acid.

The product from Example 32 was converted to the title compound following the procedure described in Example 30. Mass spectrum: $(M)^+ = 277$.

Example 34

(2R)-3-t-Butyloxycarbonyl-2-benzylpropionic Acid.

The resultant compound from Example 26 and an equal weight of 10% palladium on carbon in methanol were stirred under a hydrogen atmosphere for 1 h. The mixture was filtered and evaporated to provide the desired product. $^1$H NMR ($CDCl_3$, TMS) $\delta$ 7.25 (m,5H), 3.07 (m,2H), 2.73 (dd,1H), 2.56 (dd,1H), 2.33 (dd,1H), 1.40 (s,9H).

Example 35

Boc-Phe-dl-3-pyrazolylalanine Methyl Ester.

To dl-3-pyrazolylalanine methyl ester dihydrochloride (dl connotes 50/50 mixture of dextrorotatory/levorotatory) (2.05 g, 8.5 mmol) in dimethylformamide (10 ml) at -10° C was added Boc-Phe N-hydroxysuccinimide ester (2.50 g, 6.90 mmol) and N-methylmorpholine (2.8 ml, 25 mmol). The mixture was stirred at -10° C for 1 h and then at 25° C for 12 h. The mixture was partitioned between ethyl acetate and saturated $NaHCO_3$ solution, and extracted with ethyl acetate which was washed with water, dried over

$Na_2SO_4$ and evaporated to afford 2.75 g (95%) of the desired product.
Anal. Calcd. for $C_{21}H_{28}N_4 \cdot 0.25\ H_2O$: C, 59.92; H, 6.82; N, 13.31.
Found: C, 59.82; H, 6.75; N, 13.13.

Example 36

Boc-Phe-dl-3-pyrazolylalanine

Boc-Phe-dl-3-pyrazolyalanine methyl ester (0.210 g, 0.505 mmol) in dioxane (1.5 ml) and water (1.0 ml) was treated with lithium hydroxide monohydrate (0.0272 g, 0.648 mmol), stirred at 25° C for 30 min and quenched with 0.32 ml 2 M HCl. The mixture was poured into chloroform, washed with water, dried over $Na_2SO_4$ and evaporated to afford 0.184 g (91%) of the desired compound.
Anal. Calcd. for $C_{20}H_{26}N4O5.0.25\ H_2O$: C, 59.03; H, 6.56; N, 13.77.
Found: C, 58.66; H, 6.70; N, 13.65.

Example 37

α-Isocyanato-L-(O-methyl)tyrosine.

A suspension of (O-methyl)tyrosine methyl ester hydrochloride (6 g) in toluene (125 ml) was heated at 100° C while phosgene was bubbled into the reaction mixture. After 2 h the mixture became homogeneous and the phosgene was continued for an additional 15 min. The mixture was cooled and evaporated with several benzene chasers to provide the desired product.

Example 38

1-Benzyloxycarbonylamino-2,3-propanediol.

1-Amino-2,3-propanediol (15.2 g, 167 mmol) and NaOH (8.1 g, 204 mmol) in water (70 ml) at -10° C was treated dropwise with benzyl chloroformate (28.5 ml, 200 mmol) in ether (30 ml) over 20 min. The reaction was stirred at 0° C for 30 min then at room temperature for 2 h. The mixture was acidified with 2 M HCl and extracted with ethyl acetate which was washed with 0.5 M $H_3PO_4$ and brine, then dried over $Na_2SO_4$ and evaporated. Recrystallization of the residue from benzene afforded 16.59 g (44%) of the desired product as a white powder. NMR (300 MHz, $CD_3OD$, ppm): 3.12 (dd,1H), 3.28 (dd,1H), 3.50 (m,2H), 3.68 (m,1H), 5.08 (s,2H), 7.35 (m,5H).

Example 39

1-Methylamino-2,3-propanediol.

Lithium aluminum hydride (7.20 g, 189 mmol) in tetrahydrofuran (THF, 300 ml) was heated to reflux and the resultant compound from Example 38 (17.0 g, 75.5 mmol) in THF (150 ml) was added dropwise over 10

min. The mixture was refluxed for 2 h, cooled, quenched sequentially with water (10 ml), 3 M NaOH (40 ml) and water (20 ml), then filtered and concentrated. The residue was dissolved in water which was washed with ether and evaporated. Bulb to bulb distillation of the residue afforded 2.0 g (25%) of the desired compound as an oil. NMR (300 MHz, $CDCl_3$, ppm): 2.45 (s,3H), 2.68 (dd,1H), 2.77 (dd,1H), 3.61 (dd,1H), 3.72 (dd,1H), 3.78 (m,1H).

Example 40

(N-Methyl-2,3-dihydroxypropylamino)carbonyl-(O-methyl)tyrosine Methyl Ester.

To the resultant compound from Example 37 (1.53 g, 6.5 mmol) in dioxane (5 ml) at 0° C was added the resultant compound from Example 39 (0.684 g, 6.5 mmol). The reaction was stirred at 0° C for 1 h then at room temperature for 1 h, evaporated and chromatographed on silica gel with 5% methanol in chloroform to afford 1.855 g (84%) of the desired product as an oil. NMR (300 MHz, $CDCl_3$, ppm), 2.88, 2.89 (s,3H total), 3.05 (m,2H), 3.26-3.60 (m,5H), 3.73 (s,3H), 3.80 (s,3H), 4.70 (m,1H), 5.07 (broad t,1H), 6.83 (dd,1H), 7.02 (dd,1H).

Example 41

(N-Methyl-2,3-dihydroxypropylamino)carbonyl-(O-methyl)tyrosine.

The resultant compound from Example 40 (114 mg, 0.355 mmol) in dioxane (4 ml) and water (2 ml) at 0° C was treated with LiOH monohydrate (42.0 mg, 1 mmol). After 90 min 2 M HCl (0.6 ml, 1.2 mmol) was added and the mixture was evaporated to a foam which was used without further purification; DCI-MS: $(M+H)^+$ = 327.

Example 42

3,3-Dimethylglutaric Acid Mono t-Butyl Ester.

3,3-Dimethylglutaric anhydride (455 mg, 3.2 mmol) in tetrahydrofuran (THF, 5 ml) was treated with sublimed potassium t-butoxide (395 mg, 3.5 mmol). After 30 min the solution was concentrated, poured into saturated $NaHCO_3$ solution and washed with ether. The aqueous phase was acidified to pH 4 with 0.5 M $H_3PO_4$ and extracted with chloroform which was dried over $Na_2SO_4$ and evaporated to afford 179 mg (26%) of the desired product as an oil. NMR (300 MHz, $CDCl_3$, ppm), 1.13 (s,6H), 1.47 (s,9H), 2.33 (s,2H), 2.45 (s,2H).

Example 43

(4-t-Butyloxycarbonyl-3,3-dimethyl)butanoylphenylalanine Benzyl Ester

Prepared according to the mixed anhydride procedure from the resultant compound from Example 42

and phenylalanine benzyl ester p-toluenesulfonic acid salt. NMR (300 MHz, $CDCl_3$, ppm), 0.96 (s,3H), 1.00 (s,3H), 1.44 (s,9H), 1.90 (d,1H), 2.16 (d,1H), 2.25 (d,1H), 2.29 (d,1H), 3.03 (dd,1H), 3.17 (dd,1H), 4.92 (m,1H), 5.12 (d,1H), 5.16 (d,1H), 7.10-7.40 (m,10H).

## Example 44

### (4-t-Butyloxycarbonyl-3,3-dimethyl)butanoylphenylalanine.

Using the procedure of Example 34 with the resultant compound of Example 43 gave the desired product as an oil. NMR (300 MHz, $CDCl_3$, ppm), 0.93 (s,3H), 0.99 (s,3H), 1.45 (s,9H), 1.77 (d,1H), 2.10 (d,1H), 2.19 (d,1H), 2.25 (d,1H), 3.02 (dd,1H), 3.33 (dd,1H), 4.72 (m,1H), 7.25 (m,5H).

## Example 45

### 2(R,S)-(4-morpholinylcarbonylmethyl)-3-(1′-naphthyl)-propionic Acid.

To 50 ml of absolute ethanol was added 2 g of sodium metal. The suspension was stirred vigorously until all the sodium dissolved and the evolution of hydrogen ceased. To this solution of sodium ethoxide was added a solution of 116 g of diethylsuccinate in 10.4 g of 1-naphthaldehyde. The solution was heated to reflux for 3 h, at which time it was cooled to room temperature and concentrated. The residue was dissolved in 320 ml of water and extracted 6 times with 100 ml portions of ether. The aqueous layer was acidified with 2N HCl and extracted with 2 x 300 ml of ether and dried with anhydrous magnesium sulfate. Evaporation of the solvent gave a yellow gummy solid which was hydrogenated to the saturated acid using Pd/C as catalyst. Coupling of the resulting saturated acid to morpholine using the mixed anhydride method followed by ester hydrolysis using the procedure of Example 36 gave the desired acid. Mass spectrum: $(M+H)^+$ = 328.

## Example 46

### ((4-Thiomorpholinyl)carbonyl)-Phe Methyl Ester.

A suspension of L-phenylalanine methyl ester hydrochloride (6 g) in toluene (125 ml) was heated to 100° C and phosgene gas was bubbled into the reaction mixture. After approximately 1.5 h, the mixture became homo genous. The bubbling of phosgene was continued for 10 more min. The solvent was then evaporated and the residue chased with benzene several times. The residue was then dissolved in ~100 ml of methylene chloride and cooled to ~0° C, and 1.1 equivalent of thiomorpholine was added dropwise. After 10 min the solution was washed with 1N HCl and the organic layer was dried with $MgSO_4$. Evaporation of solvent gave 5.5 g of product. Mass spectrum: $M^+$ = 308.

## Example 47

### ((4-Sulphonylmorpholinyl)carbonyl)-Phe Methyl Ester.

To 2 g of the product from Example 46 in 100 ml of methylene chloride was added 2.94 g of metachloroperbenzoic acid at 0 °C. After 30 min the solvent was evaporated, dissolved in ether, and the ether solution was washed with 10% sodium sulfite solution and then with saturated sodium bicarbonate several times. The organic layer was dried with $MgSO_4$ and evaporation of the solvent gave a white solid which was purified by silica gel column chromatography (20% EtOAc/80%/$CH_2Cl_2$) to give 2.10 g (95%) of pure product. Mass spectrum: $M^+$ = 340.

Example 48

((4-sulfonylmorpholinyl)carbonyl)-Phe-OH.

Using the procedure described in Example 19, with the resultant compound from Example 47 gave the desired compound.

Example 49

Morpholinocarbonyl-(O-methyl)tyrosine Methyl Ester.

To the resultant compound from Example 37 in methylene chloride at 0 °C was added one equivalent of morpholine. After 1 h the solvent was evaporated and the residue was chromatographed on silica gel with ethyl acetate/hexane mixtures to provide the desired product.

Example 50

Morpholinocarbonyl-(O-methyl)tyrosine.

Using the procedure from Example 19 with the resultant compound from Example 49 gave the desired product.
Anal. Calcd. for $C_{15}H_{20}N_2O_5 \cdot 0.25\ H_2O$: C, 57.59; H, 6.66; N, 8.95.
Found: C, 57.79; H, 6.57; N, 8.93.

Example 51

Phenethylmethylaminocarbonyl-(O-methyl)tyrosine Methyl Ester.

Prepared according to the procedure of Example 49 replacing morpholine with N-methyl-2-phenylethylamine. $^1H$ NMR ($CDCl_3$, TMS) $\delta$ 7.25 (m,5H), 7.02 (m,2H), 6.82 (m,2H), 4.73 (m,2H), 3.78 (s,3H), 3.73 (s,3H), 3.45 (m,2H), 3.03 (d,2H), 2.81 (d,2H), 2.77 (s,3H).

Example 52

Phenethylmethylaminocarbonyl-(0-methyl)tyrosine.

Using the procedure from Example 19 with the resultant compound from Example 51 gave the desired product. $^1$H NMR ($CDCl_3$, TMS) d 7.28 (m,3H), 7.10 (m,4H), 6.82 (m,2H), 4,40 (m,2H), 3.78 (s,3H), 3.38 (m,2H), 2.70 (s,3H).

Example 53

(3,4-cis-Dihydroxypyrrolidinylcarbonyl)-(O-methyl)tyrosine Methyl Ester.

A suspension of L-(O-methyl)tyrosine methyl ester hydrochloride (10 g) in toluene (~200 ml) was heated to 100 °C while phosgene gas was bubbled into the reaction mixture. After approximately 2 h the mixture became homogeneous. The bubbling of phosphene was continued for 15 more minutes keeping the temperature at ~100 °C. The toluene was then evaporated and the residue chased with benzene several times. The isocyanate from L-(Me)Tyr-$OCH_3$ was then dissolved in ~100 ml of methylene chloride and 1.1 equivalent of 3-pyrroline (75% pure) was added dropwise at 0 °C. After 15 min, the reaction mixture was washed with 0.5 N HCl and methylene chloride. The organic layer was washed with aqueous $NaHCO_3$ and dried over $MgSO_4$. Evaporation of the solvent gave 3-pyrrolinylcarbonyl-(Me)Tyr-methyl ester which was cis-hydroxylated under the following conditions: 2.5 g of the 3-pyrrolinylcarbonyl-(Me)Tyr-methyl ester which was dissolved in 50 ml of THF and 1 ml of a 2.5% solution of $OsO_4$ in t-butanol was added, followed by 1.15 g of N-methyl-morpholine-N-oxide. After 1 h, the solvent was evaporated and the residue dissolved in 150 ml of ethyl acetate and washed with dilute $Na_2SO_3$ solution, saturated $NaHCO_3$ solution and then dried with $MgSO_4$. Evaporation of the solvent gave a gummy solid which was purified by $SiO_2$ column chromatography (5% MeOH/$CH_2Cl_2$) to give the desired compound (65% yield). Mass spectrum: M+ = 338.

Example 54

(3,4-cis-Dihydroxypyrrolidinylcarbonyl)-(O-methyl)tyrosine.

Using the procedure from Example 19 with the resultant compound from Example 53 and replacing ether extractions with chloroform extractions gave the desired product. Mass spectrum: $M^+$ = 324.

Example 55

1-Benzyloxycarbonyl-3,4-cis-dihydroxypyrrolidine.

3-Pyrroline (75% pure) was reacted with benzyl chloroformate and the resulting product was cis-hydroxylated according to the procedure of Example 53 to give the desired product.

Example 56

1-Benzyloxycarbonyl-3,4-cis-dimethoxymethoxypyrrolidine.

Reaction of MOM-Cl in the presence of diisopropylethyl amine with the resultant compound from Example 55 gave the desired product. $^1$H NMR ($CDCl_3$, TMS) δ 7.35 (m,5H), 5.16 (d,1H), 5.13 (d,1H), 4.73 (m,4H), 4.21 (m,2H), 3.60 (m,4H), 3.40 (s,3H), 3.38 (s,3H).

Example 57

Cis-3,4-dimethoxymethoxypyrrolidine.

Hydrogenolysis of the resultant compound from Example 56 gave the desired product. $^1$H NMR ($CDCl_3$, TMS) δ 4.71 (m,4H), 4.12 (m,2H), 3.38 (s,6H), 3.09 (m,2H), 2.90 (m,2H).

Example 58

Benzyl (2R)-2-Benzyl-3-(3,4-cis-dimethoxymethoxypyrrolidinocarbonyl) propionate.

Using the mixed anhydride procedure with the resultant compounds from Example 27 and Example 57 gave the desired product. $^1$H NMR ($CDCl_3$, TMS) δ 7.10-7.35 (m,10H), 5.10 (m,2H), 4.72 (m,4H), 4.18 (m,2H), 3.37 (m,6H), 3.05 (m,1H), 2.82 (m,1H), 2.62 (m,1H), 2.30 (m,1H).

Example 59

(2R)-2-Benzyl-3-(3,4-cis-dimethoxymethoxypyrrolidinocarbonyl)propionic Acid.

Using the procedure of Example 30 with the resultant compound from Example 58 gave the desired product. $^1$H NMR ($CDCl_3$, TMS) δ 7.26 (m,5H), 4.70 (m,4H), 4.20 (m,2H), 2.75 (dd,1H).

Example 60

Boc-Phe-Leu amide of 2(S)-Amino-1-cyclohexyl-4,4-difluoro-3(R),5(R)dihydroxy-6-methylheptane.

Boc-Phe-Leu-OH was coupled to the resultant product from Example 9 using the mixed anhydride procedure described below: To 82 mg of Boc-Phe-Leu-OH in 5 ml of THF at -20° C was added 1 equivalent of N-methylmorpholine, followed by 1 equivalent of isobutyl chloroformate. The mixture was stirred for 10 min, a solution of the product from Example 9 (50 mg in 5 ml THF) was added. The mixture was stirred for 0.5 hr, filtered and concentrated. The residue was dissolved in 50 ml of ethyl acetate and washed successively with satd. $NaHCO_3$, dilute HCl and then brine. The organic layer was dried and evaporated. The residue was chromatographed on silica gel (20% EtOAc in $CH_2Cl_2$) to give 88 mg of product. $^1$H NMR ($CDCl_3$) δ 0.91 (d,3H, J=3Hz), 0.93 (d,3H, J=3Hz), 1.04 (d,6H, J=6.5 Hz), 1.42 (s,9H), 6.28 (br d,1H), 6.82 (br d,1H), 7.2-7.36 (m,5H). Anal. Calcd. for $C_{34}H_{55}N_3O_6F_2$; C, 63.82; H, 8.66; N, 6.57.

Found: C, 63.57; H, 8.61; N, 6.37.

Example 61

Boc-Phe-Leu amide of 2(S)-Amino-1-cyclohexyl-4,4-difluoro-3(R),5(S)-dihydroxy-6-methylheptane.

Using the same procedure described in Example 60, replacing the resultant compound from Example 9 with that from Example 10 provided the desired compound. FAB MS: $(M+H)^+$ = 640.

Example 62

Benzyl (2R)-2-Benzyl-3-(4-trifluoroethylpiperazin-1-ylcarbonyl)propionate.

The resultant acid from Example 27 (0.500 g, 1.68 mmol) in $CH_2Cl_2$ (7 ml) at -10° C was treated with N-methylmorpholine (0.20 ml, 1.82 mmol) and then isobutyl chloroformate (0.22 ml, 1.68 mmol). After 5 min 1-trifluoroethylpiperazine (0.30 g, 1.78 mmol) was added and the mixture was stirred at -10° C for 15 min and then at room temperature for 2 h. The solvent was evaporated and the residue was taken up in ethyl acetate which was washed with saturated $NaHCO_3$ solution, water and brine, and then dried over $Na_2SO_4$ and evaporated. Chromatography of the residue on silica gel with 20-33% ethyl acetate in hexane provided 0.61 g (81%) of an oil. $^1H$ NMR ($CDCl_3$) $\delta$ 7.10- 7.40 (m,10H), 5.15 (d,1H), 5.05 (d,1H), 3.25-3.70 (m,5H), 3.04 (dd,1H), 2.97 (q,2H), 2.81 (dd,1H), 2.72 (dd,1H), 2.60 (m,4H), 2.32 (dd,1H).

Example 63

(2R)-2-Benzyl-3-(4-trifluoroethylpiperazin-1-ylcarbonyl)propionic Acid.

The resultant compound from Example 62 (610 mg), and 10% palladium on carbon (300 mg) in methanol were stirred under an $H_2$ atmosphere for 2 h. Filtration and solvent evaporation afforded 470 mg (96%) of a solid, m.p. 96-98° C.

Example 64

Benzyl (2R)-2-Benzyl-3-(4-methylpiperazin-1-ylcarbonyl)propionate.

Using the procedure of Example 62 but replacing 1-trifluoroethylpiperazine with 1-methylpiperazine provided the desired product as an oil. $^1H$ NMR ($CDCl_3$) $\delta$ 7.10-7.40 (m,10H), 5.16 (d,1H), 5.05 (d,1H), 3.25-3.70 (m,5H), 3.03 (dd,1H), 2.82 (dd,1H), 2.72 (dd,1H), 2.35 (m,5H), 2.28 (s,3H).

Example 65

(2R)-2-Benzyl-3-(4-methylpiperazin-1-ylcarbonyl)propionic Acid.

Prepared from the resultant compound of Example 64 according to the procedure of Example 63. $^1H$ NMR ($CDCl_3$) $\delta$ 7.15-7.35 (m,6H), 2.50-2.80 (m,4H), 2.47 (s,3H), 2.30 (dd,1H).

Example 66

Benzyl (2R)-2-Benzyl-3-((2-pyridin-2-ylethyl)methylaminocarbonyl)propionate.

Using the procedure of Example 62 but replacing 1-trifluoroethylpiperazine with 2-(2-methylaminoethyl)-pyridine provided the desired product as an oil. $^1H$ NMR ($CDCl_3$) $\delta$ 8.48 (m,1H), 7.57 (m,1H), 6.95-7.40 (m,12H), 5.00-5.20 (m,2H), 2.87 2.82 (2s,total 3H), 2.31, 2.18 (2dd,total 1H).

Example 67

(2R)-2-Benzyl-3-((2-pyridin-2-ylethyl)methylaminocarbonyl)propionic Acid.

Prepared from the resultant compound of Example 66 according to the procedure of Example 63. $^1H$ NMR ($CDCl_3$) $\delta$ 8.49 (m,1H), 7.58 (m,1H), 6.95-7.32 (m,7H), 2.87, 2.72 (2s,total 3H).

Example 68

Boc-L-(4-thiazolyl)alanine amide of 2(S)Amino-1-cyclohexyl-4,4-difluoro-3(R),5(R)-dihydroxy-6-methylheptane.

Using the procedure described in Example 14, but replacing Boc-His-OH with Boc-L-(4-thiazolyl)alanine provided the desired product. FAB Mass spectrum: $(M+H)^+$ = 523.

Example 59

Benzyl (2R)-2-Benzyl-3-chloromethylcarbonylpropionate.

The resultant acid from Example 27 (500 mg, 1.68 mmol) in $CH_2Cl_2$ (8 ml) at 0°C was treated with oxalyl chloride (0.160 ml, 1.83 mmol) and dimethylformamide (0.0065 ml). After 2 h at 0°C, the solvent was evaporated and the residue was dissolved in ether (6 ml), cooled to 0°C and treated with an ether solution of $CH_2N_2$. After 2 h at 0°C the solvent was evaporated and the residue was dissolved in ether (6 ml), cooled to -10°C, and treated with 4.0 M HCl/dioxane (0.6 ml, 2.4 mmol). After 1 h the solvent was evaporated and the residue was chromatographed on silica gel.

Example 70

Benzyl (2R)-2-Benzyl-5-tert-butylmercapto-4-oxopentanoate.

To tert-butylmercaptan (0.11 ml) in dimethylformamide (5 ml) at 0 °C was added potassium bis-(trimethylsilyl)amide in toluene (1.80 ml, 0.90 mmol, 0.5 M ) followed by the resultant compound from Example 69 (259.6 mg, 0.785 mmol) in dimethylformamide (3 ml). After 16 h at room temperature the mixture was diluted with ethyl acetate, washed with water and brine, then dried over $Na_2SO_4$ and evaporated. Chromatography of the residue on silica gel with 10% ethyl acetate in hexane afforded 219.6 mg (73%) of a colorless oil. $^1H$ NMR ($CDCl_3$) $\delta$ 7.10-7.40 (m,10H), 5.07 (s,2H), 3.25 (s,2H), 3.18-3.29) (m,1H), 2.97-3.07 (m,2H), 2.78 (dd,1H), 2.71 (dd,1H), 1.25 (s,9H).

## Example 71

Benzyl (2R)-2-Benzyl-5-tert-butylsulfonyl-4-oxopentanoate.

The resultant compound from Example 70 (171.9 mg, 0.447 mmol) in $CH_2Cl_2$ (5 ml) was treated with metachloroperbenzoic acid (290 mg, 1.34 mmol, 80% pure). After 75 min at room temperature the product was isolated to afford 184.0 mg (59%) of a colorless oil. $^1H$ NMR ($CDCl_3$) $\delta$ 7.08-7.35 (m,10H), 5.07 (s,2H), 3.98 (d,1H), 3.88 (d,1H), 3.23-3.33 (m,1H), 3.18 (dd,1H), 3.03 (dd,1H), 2.88 (dd,1H), 2.82 (dd,1H), 1.38 (s,9H).

## Example 72

(2R)-2-Benzyl-5-tert-butylsulfonyl-4-oxopentanoic Acid.

Prepared from the resultant compound from Example 71 according to the procedure of Example 63. $^1H$ NMR ($CDCl_3$) $\delta$ 7.15-7.35 (m,5H), 3.94 (d,1H), 3.88 (d,1H), 2.90-3.30, (m,3H), 2.70-2.85 (m,2H), 1.39 (s,9H).

## Example 73

Benzyl (2R)-2-Benzyl-5-morpholin-4-yl-4-oxopentanoate.

The resultant compound from Example 69 (610 mg, 1.84 mmol) in dimethylformamide (10 ml) was treated with NaI (33 mg, 0.22 mmol) and morpholine (0.60 ml, 6.88 mmol). After 2 h the mixture was diluted with ethyl acetate, washed with water and brine, and then dried over $Na_2SO_4$, and evaporated. Chromatography of the residue on silica gel with 60% ethyl acetate/40% hexane afforded 460 mg (65%) of an oil. $^1H$ NMR ($CDCl_3$) $\delta$ 7.05-7.40 (m,10H), 5.11 (d,1H), 5.06 (d,1H), 3.68 (m,4H), 3.22-3.32 (m,1H), 3.15 (d,1H), 3.08 (d,1H), 3.05 (m,1H), 2.87 (dd,1h), 2.77 (dd,1H), 2.35-2.50 (m,5H).

## Example 74

(2R)-2-Benzyl-5-morpholin-4-yl-4-oxopentanoic Acid.

Prepared from the resultant compound from Example 73 according to the procedure of Example 63. $^1H$ NMR ($CDCl_3$) δ 7.17-7.35 (m,5H), 3.60-3.75 (m,4H).

Example 75

Benzyl(2R)-2-benzyl-3-((2-imidazol-1-ylethyl)methylaminocarbonyl)propionate.

To benzyl (2R)-2-benzyl-3-(N-(2-hydroxyethyl)-N-methyl)-aminocarbonylpropionate (110 mg, 0.31 mmole) in $CH_2Cl_2$ at -78° C was added TEA (0.07 ml, 0.5 mmole) and methanesulfonyl chloride (0.036 ml, 0.047 mmole). After 1 hr, 3 equivalents of imidazole was added and the mixture was refluxed for 4 hr. After workup and silica gel chromatography (0.5% MeOH in $CH_2Cl_2$) the desired product was obtained in 60% yield.

$^1H$ NMR ($CDCl_3$) δ 2.27 (dd,1H), 2.59 (s,3H), 2.60-2.85 (m,2h), 3.08 (m,1H), 3.32 (m,1H), 3.50 (m,1H), 3.61 (m,1H), 4.05 (m,2H), 5.08 (s,1H), 5.18 (d,1H), 6.89 (s,1H), 7.00-7.40 (m,11H), 7.42 (s,1H).

Example 76

(2R)-2-Benzyl-3-((2-imidazol-1-ylethyl)methylaminocarbonyl)propionic Acid.

Using the procedure of Example 63 with the resultant compound from Example 75 gave the desired product. $^1H$ NMR ($CDCl_3$) δ 7.83 (s,1H), 7.15-7.32 (m,5H), 7.14 (s,1H), 6.93 (s,1H), 3.30 (m,2H), 3.09 (m,1H), 2.60-2.78 (m,2H), 2.60 (s,3H).

Example 77

Boc-Phe-His-amide of 2(S)-Amino-1-cyclohexyl-4,4-difluoro-3(R),5(R)-dihydroxy-6-methylheptane.

The protecting group of the resultant compound from Example 14 was removed using 4 N HCl in dioxane. The resulting amine hydrochloride was coupled to Boc-Phe-OH using the procedure of Example 14 to provide the desired product.

Anal. Calcd. for $C_{34}H_{51}N_5O_6F_2$: C, 61.52; H, 7.74; N, 10.55.

Found: C, 61.69; H, 7.41; N, 10.57

Example 78

Boc-Phe-His amide of 2(S)-Amino-1-cyclohexyl-4,4-difluoro-3(R),5(R,S)-dihydroxyhexane.

Using the procedure described in Example 14, Boc-Phe-His-OH was coupled to the product from Example 13 to provide the desired product.

Anal. Calcd. for $C_{32}H_{47}N_5O_6F_2$: C, 60.45; H, 7.45; N, 11.02.

Found: C, 60.21; H, 7.69; N, 10.89

Example 79

Cbz((β,β-di-Me)-β-Ala)-Phe-His amide of 2(S)-Amino-1-cyclohexyl-4,4-difluoro-3(R),5(R)-dihydroxy-6-methylheptane.

Cbz((β,β-di-Me)-β-Ala)-Phe-OH was coupled to the amine hydrochloride of the product from Example 14 using the procedure described in Example 14 to provide the desired product. FAB MS: $(M+H)^+$ = 782.

Example 80

Cbz((β,β-di-Me)-β-Ala)-(Me)Tyr-His amide of 2(S)-Amino-1-cyclohexyl-4,4-difluoro-3(R),5(R)-dihydroxy-6-methylheptane.

Using the procedure of Example 79, but replacing Cbz((β,β-di-Me)-β-Ala)-Phe-OH with Cbz ((β,β)-di-Me)-β-Ala)-(Me)Tyr-OH provided the desired compound.
Anal. Calcd. for $C_{43}H_{60}N_6O_8F_2$: C, 62.44; H, 7.31; N, 10.16.
Found: C, 62.06; H, 7.52; N, 9.80.

Example 81

2(S)-((4-Morpholinyl)carbonyl)oxy-3-phenylpropionic acid amide of His-2(S)-Amino-1-cyclohexyl-4,4-difluoro-3(R)-,5(R)-dihydroxy-6-methylheptane.

The resultant product from Example 23 was coupled to the amine hydrochloride salt of the product from Example 14 using the procedure described in Example 14 to provide the desired compound. FAB MS: $(M+H)^+$ = 678.

Example 82

2(R)-2-Benzyl-3-(morpholinocarbamoyl)-propionic acid amide of His-2(S)-Amino-1-cyclohyexyl-4,4-difluoro-3(R),5-(R)-dihydroxy-6-methylheptane.

The resultant product from Example 30 was coupled to the amine hydrochloride salt of the product from Example 14 using the carbodiimide procedure described in Example 14 to provide the desired compound. FAB MS: $(M+H)^+$ = 691.

Example 83

2(R)-2-Benzyl-3-Morpholinocarbonylpropionic acid amide of His-2(S)-Amino-1-cyclohexyl-4,4-difluoro-3(R),5(R)-dihydroxy-6-methylheptane.

The resultant compound from Example 33 was coupled to the amine hydrochloride salt of the product from Example 14 using the carbodiimide procedure described in Example 14 to provide the desired compound.
Anal. Calcd. for $C_{35}H_{51}N_5O_6F_2 \cdot 1.5\ H_2O$: C, 59.80; H, 7.74; N, 9.96.
Found: C, 59.98; H, 7.50; N, 9.67.

Example 84

Boc-Phe-dl-3-pyrazolylalanine amide of 2(S)-amino-1-cyclohexyl-4,4-difluoro-3(R),5(R)-dihydroxy-6-methylheptane.

The resultant compound from Example 36 was coupled to the resultant compound from Example 9 using the carbodiimide procedure described in Example 14 to provide the desired compound. FAB MS: $(M+H)^+$ = 664.

Example 85

(N-Methyl-2,3-dihydroxypropylamino)carbonyl-(O-methyl)tyrosine amide of His-2(S)-amino-1-cyclohexyl-4,4-difluoro-3(R),5(R)-dihydroxy-6-methylheptane.

The resultant compound from Example 41 was coupled to the amine hydrochloride salt of the product from Example 14 using the carbodiimide procedure described in Example 14 to provide the desired compound.

Example 86

2(R,S)-(4-morpholinylcarbonylmethyl)-3-(1′-naphthyl)-propionic acid amide of His-2(S)-amino-1-cyclohexyl-4,4-difluoro-3(R),5(R)-dihydroxy-6-methylheptane.

The resultant compound from Example 45 was coupled to the amine hydrochloride salt of the resultant compound from Example 14 using the carbodiimide procedure described in Example 14 to provide the desired compound. FAB MS: $(M+H)^+$ = 726.

Example 87

(E)-2-((4-Morpholinylcarbonyl)methyl)cinnamic acid amide of His-2(S)-amino-1-cyclohexyl-4,4-difluoro-3(R),5(R)-dihydroxy-6-methylheptane.

The resultant compound from Example 16 was coupled to the amine hydrochloride salt of the resultant compound from Example 14 using the carbodiimide procedure described in Example 14 to provide the

desired compund. FAB MS: $(M+H)^+$ = 674.

Example 88

((4-Sulfonylmorpholinyl)carbonyl)-Phe-His amide of 2(S)-amino-1-cyclohexyl-4,4-difluoro-3(R),5(R)-dihydroxy-6-methylheptane.

The resultant compound from Example 48 was coupled to the amine hydrochloride salt of the resultant compound from Example 14 using the carbodiimide procedure described in Example 14 to provide the desired compound. FAB MS: $(M+H)^+$ = 725.

Example 89

Phenethylmethylaminocarbonyl-(O-methyl)tyrosine-His amide of 2(S)-amino-1-cyclohexyl-4,4-difluoro-3(R),5(R)-dihydroxy-6-methylheptane.

The resultant compound from Example 52 was coupled to the amine hydrochloride salt of the resultant compuond from Example 14 using the carbodiimide procedure described in Example 14 to provide the desired compound.

Example 90

(3,4-cis-Dihydroxypyrrolidinylcarbonyl)-(O-Methyl)tyrosine-His amide of 2(S)-amino-1-cyclohexyl-4,4-difluoro-3-(R),5(R)-dihydroxy-6-methylheptane.

The resultant compound from Example 54 was coupled to the amine hydrochloride salt of the resultant compound from Example 14 using the carbodiimide procedure described in Example 14 to provide the desired compound. FAB MS: $(M+H)^+$ = 723.

Example 91

2(R)-2-Benzyl-3-(3,4-cis-dimethoxymethoxypyrrolidinocarbonyl)propionic acid amid of His-2(S)-amino-1-cyclohexyl-4,4-difluoro-3(R),5(R)-dihydroxy-6-methylheptane.

The resultant compound from Example 59 was coupled to the amine hydrochloride salt of the resultant compound from Example 14 using the carbodiimide procedure described in Example 14 to provide the desired compound. FAB MS: $(M+H)^+$ = 780.

Example 92

Boc-Phe-L-(4-thiazolyl)alanine amide of 2(S)-amino-1-cyclohexyl-4,4-difluoro-3(R),5(R)-dihydroxy-6-methylheptane.

The resultant compound from Example 68 was deprotected with 4 N HCl in dioxane and the resultant amine hydrochloride was coupled to Boc-Phe-OH using the carbodiimide procedure described in Example 14 to provide the desired compound. FAB MS: $(M+H)^+ = 682$.

## Example 93

2(R)-2-Benzyl-3(4-methylpiperazine-1-ylcarbonyl)propionic acid amide of L- (4-thiazolyl)alamine-2(S) amino-1-cyclohexyl-4,4-difluoro-3(R),5(R)-dihydroxy-6-methylheptane.

The resultant compound from Example 65 was coupled to the amine hydrochloride salt of the resultant compound from Example 68 using the procedure described in Example 14 to provide the desired compound.

Anal. Calcd. for $C_{36}H_{53}N_5O_5F_2S \cdot 0.25\ H2O$: C, 60.87; H, 7.59; N, 9.86.

Found: C, 60.65; H, 7.59; N, 9.75.

## Example 94

2(R)-2-Benzyl-3-(4-trifluoroethylpiperazine-1-ylcarbonyl)propionic acid amide of His-2(S)-amino-1-cyclohexyl-4,4-difluoro-3(R),5(R)-dihydroxy-6-methylheptane.

The resultant compound from Example 63 was coupled to the amine hydrochloride salt of the resultant compound from Example 14 using the carbodiimide procedure described in Example 14 to provide the desired compound.

## Example 95

2(R)-2-Benzyl-3-((2-pyridin-2-ylethyl)methylaminocarbonyl)propionic acid amide of His-2(S)-amino-1-cyclohexyl-4,4-difluoro-3(R),5(R)-dihydroxy-6-methylheptane.

The resultant compound from Example 67 was coupled to the amine hydrochloride salt of the resultant compound from Example 14 using the carbodiimide procedure described in Example 14 to provide the resultant compound. FAB MS: $(M+H)^+ = 709$.

## Example 96

2(R)-2-Benzyl-5-tert-butysulfonyl-4-oxopentanoic acid amide of His-2(S)-amino-1-cyclohexyl-4,4-difluoro-3(R),5-(R)-dihydroxy-6-methylheptane.

The resultant compound from Example 72 was coupled to the amine hydrochloride salt of the resultant compound from Example 14 using the carbodiimide procedure described in Example 14 to provide the desired compound.

## Example 97

### 2(R)-2-Benzyl-3-((2-imidazolylethyl)methylaminocarbonyl)propionic acid amide of L-(4-thiazolyl)alanine-2(S)-amino-1-cyclohexyl-4,4-difluoro-3(R),5(R)-)-dihydroxy-6-methylheptane.

The resultant compound from Example 76 was coupled to the amine hydrochloride of the resultant compound from Example 68 using the procedure described in Example 14 to provide the desired compound.
Anal. Calc. for $C_{37}H_{52}N_6O_5F_2S \cdot 0.5\ H_2O$: C, 60.06; H, 7.22; N, 11.36.
Found: C, 60.09; H, 7.08; N, 11.20.

## Example 98

### Boc-Phe-Leu amide of 2(S)-Amino-1-cyclohexyl-4,4-difluoro-3,5-dioxo-6-methylheptane.

The resultant product from Example 60 was oxidized to the dioxo compound using excess Dess-Martin's periodinane (J. Org. Chem. 48 , 4155-4156, 1983) in $CH_2Cl_2$ to provide the desired compounds.
FAB MS: $(M+H)^+ = 636$.

## Example 99

### N-Methyl-N-[2-(2-pyridylethyl)]-3(S)-acetoxy-2(R)-benzylsuccinamide

To a solution of 1.1 g (3.8 mmol) of diethyl (2R,3S)-2-benzyl-3-hydroxy succinate, prepared according to the procedure of D. Seebach, Org. Syn., **63**, 109, in 16 mL of tetrahydrofuran, cooled in an ice-water bath was added 440 mg (10.4 mmol) of lithium hydroxide monohydrate in 16 mL water. The bath was removed and the reaction stirred for 18 h. The reaction mixtured was acidified with concentrated HCl until pH 4 and the solvents removed under high vacuum to give a white solid. The crude diacid was warmed to 50° C in 8 mL of 1:1 acetic anhydride/acetyl chloride for 3 h. Excess acetic anhydride/acetyl chloride was removed under high vacuum. The residue is dissolved in 5 mL of methylene chloride, cooled in an ice-water bath and 0.51 g (3.8 mmol) of 2-(2-methylamminoethyl)pyridine is added. The reaction is stirred for 1 h and the solvents removed under high vacuum. The crude acid is used without further purification.

## Example 100

### (2S, 3R)-Benzyl-3-[2-(2-pyridylethyl)methylaminocarboyl]-3-hydroxypropionic Acid Lithium Salt

A solution of 0.16 g (0.45 mmol) of ester from Example 137 in 3 mL of tetrahydrofuran is treated with

21 mg (0.5 mmol) of lithium hydroxide monohydrate in 3 mL of water. The reaction is stirred at room temperature for 18 h. The solvents are removed under high vacuum to give a solid lithium salt which is used without further purification.

Example 101

(2R,3S)-3-Acetoxy-2-Benzyl-3-(2-(2-pyridylethyl)methylaminocarbonyl)propionyl-L-(4-thiazolyl)Ala Amide of (2S)-Amino-1-cyclohexyl-4,4-difluoro-3(R),5(R)-dihydroxy-6-methylheptane

Using the coupling procedure of Example 14, the resultant compound of Example 99 is coupled to the amine hydrochloride of the resultant compound of Example 15 to provide the desired product.

Example 102

(2R,3S)-2-Benzyl-3-hydroxy-3-(2-pyridylethyl)methylaminocarbonyl)propionyl-L-(4-thiazolyl)Ala Amide of (2S)-Amino-1-cyclohexyl-4,4-difluoro-3(R),5(R)-dihydroxy-6-methylheptane

Using the coupling procedure of Example 14, the resultant compound of Example 100 is coupled to the amine hydrochloride of the resultant compound of Example 15 to provide the desired compound.

Example 103

Benzyl (2R)-2-Benzyl-3-[(2-pyrazol-1-ylethyl)methylaminocarbonyl]propionate

Using the procedure of Example 75 and replacing imidazole with pyrazole gave, after chromatography on silica gel with 0.5% methanol in chloroform, the desired product. $^{1}H$ NMR ($CDCl_3$) $\delta$ 7.51, 7.40 (2d, total 1H), 7.18-7.38(m,10H), 7.08-7.17(m,1H), 6.21, 6.13(2dd, total 1H), 4.99-5.20(4d, total 2H), 2.78, 2.52(2S, total 3H).

Example 104

(2R)-2-Benzyl-3-[(2-pyrazol-1-ylethyl)methylaminocarbonyl]propionic Acid

Using the procedure of Example 63 with the resultant compound from Example 103 gave the desired product. $^{1}H$ NMR ($CDCl_3$) $\delta$ 7.62(d,1H), 7.51, 7.45(2d, total 1H), 7.15-7.37(m,5H), 6.22, 6.19(2dd, total 1H), 2.82, 2.49(2s, total 3H).

Example 105

(2R)-2-Benzyl-3-[(2-pyrazol-1-ylethyl)methylaminocarbonyl]propionic acid amide of L-(4-thiazolyl)alanine-2(S)-amino-1-cyclohexyl-4,4-difluoro-3(R),5(R)-dihydroxy-6-methylheptane

The resultant compound from Example 104 was coupled to the amine hydrochloride of the resultant compound from Example 68 using the procedure described in Example 14 to provide the desired compound.$^1$H NMR ($CDCl_3$) δ 8.75, 8.73 (2 d, total 1H), 8.41, 8.22 (2 d, total 1H), 7.52-7.44 (m, 2H), 6.98, 6.83 (2 d, total 1H), 6.25-6.21 (m, 1H), 2.80, 2.46 (2 s, total 3H).

Example 106

Benzyl (2R)-2-benzyl-3-((2-diethylaminoethyl)methylaminocarbonyl)propionate.

Using the procedure of Example 62 but replacing 1-trifluoroethylpiperazine with N,N-diethyl-N′-methylethylene diamine gave, after chromatography on silica gel with 3% methanol in chloroform, the desired product.$^1$H NMR ($CDCl_3$) δ 7.09-7.37 (m,10H), 5.02-5.20 (m,2H), 2.94, 2.88 (2s,total 3H), 0.91-1.08 (m,6H).

Example 107

(2R)-2-Benzyl-3-((2-diethylaminoethyl)methylaminocarbonyl)propionic Acid.

Using the procedure of Example 63 with the resultant product from Example 106 gave the desired product. $^1$H NMR ($CDCl_3$) δ 7.12-7.30 (m,5H), 3.00 (s,3H), 1.17 (t,6H).

Example 108

Benzyl (2R)-2-Benzyl-3-thiazol-4-ylpropionate.

The resultant compound from Example 69 (476.8 mg, 1.44 mmol) and thioformamide (176 mg, 2.88 mmol) in acetone (6 ml) were stirred at room temperature for 108 h. N-methylmorpholine (0.16 ml, 1.40 mmol) was added and after 20 min the mixture was diluted with ether, filtered, evaporated, and chromatographed on silica gel with 20% ethyl acetate in hexane to afford 369 mg (76%) of an oil. $^1$H NMR ($CDCl_3$) δ 8.70 (d,1H), 7.05-7.35 (m,10H), 6.90 (d,1H), 5.00 (d,1H), 4.95 (d,1H), 3.28-3.35 (m,1H), 3.19 (dd,1H), 2.95-3.10O (m,2H), 2.88 (dd,1H).

Example 109

(2R)-2-Benzyl-3-thiazol-4-ylpropionic Acid

The resultant compound from Example 108 (364 mg) was stirred for 2 h in 30% HBr in acetic acid (5 ml). The solvent was evaporated and the residue was dissolved in 1 M HCl and washed with ether. The

aqueous phase was adjusted to pH 4 with solid $NaHCO_3$ and extracted with chloroform which was dried over $Na_2SO_4$ and evaporated to afford 186.5 mg (70%) of an oil. $^1H$ NMR ($CDCl_3$) δ 8.78 (d,1H), 7.15-7.35 (m,5H), 6.99 (d,1H), 3.00-3.30 (m,4H), 2.81 (dd,1H).

Example 110

Methyl α-Benzylacrylate.

α-Benzylacrylic acid (1.00 g, 6.17 mmol) in methanol (20 ml) was treated with $BF_3 \cdot Et_2O$ (2 ml). The mixture was heated to reflux for 14 h, cooled, and poured into saturated $NaHCO_3$ solution. Extraction with ether followed by drying over $Na_2SO_4$ and evaporation afforded 1.03 g (95%) of a mobile oil. $^1H$ NMR ($CDCl_3$) δ 7.17-7.35 (m,5H), 6.23 (m,1H), 5.47 (m,1H), 3.74 (s,3H), 3.63 (s,2H).

Example 111

Methyl (2RS)-2-benzyl-3-(N-methoxyl-N-methylamino)propionate.

The resultant compound from Example 110 (800 mg, 4.54 mmol), N-methyl,O-methylhydroxylamine hydrochloride (0.57 g, 5.4 mmol), and $NaHCO_3$ (0.46 g, 5.48 mmol) in dimethylsulfoxide (5 ml) were heated at 130 °C for 20 h. The mixture was diluted with ethyl acetate, washed with water, saturated $NaHCO_3$ solution and brine, and then was dried over $Na_2SO_4$ and evaporated. Chromatography of the residue on silica gel with 10% ethyl acetate in hexane afforded 226 mg (21%) of a mobile oil. $^1H$ NMR ($CDCl_3$) δ 7.10-7.30 (m,5H), 3.60 (s,3H), 3.47 (s,3H), 2.80-3.10 (m,4H), 2.60 (dd,1H), 2.55 (s,3H).

Example 112

Methyl (2RS)-2-benzyl-3-pyrazol-1-ylpropionate

Using the procedure of Example 111 but replacing N-methyl,O-methylhydroxylamine hydrochloride and $NaHCO_3$ with pyrazole provided the desired product as an oil. $^1H$ NMR ($CDCl_3$) δ 7.52 (d,1H), 7.10-7.35 (m,6H), 6.10 (dd,1H), 4.38 (dd,1H), 4.24 (dd,1H), 3.57 (s,3H), 3.37 (m,1H), 2.98 (dd,1H), 2.82 (dd,1H).

Example 113

(2RS)-2-Benzyl-3-pyrazol-1-ylpropionic Acid.

The resultant compound from Example 112 (100.0 mg, 0.409 mmol) in dioxane (2 ml) at 0 °C was treated with $LiOH \cdot H_2O$ (22.0 mg, 0.524 mmol) in water (1 ml). After I h at 0 °C and 30 min at room temperature the solvent was evaporated and the residue was taken up in water, the pH was adjusted to pH 3-4, and the mixture was extracted with $CHCl_3$ which was dried over $Na_2SO_4$ and evaporated to afford 96.mg (100%) of a solid. $^1H$ NMR ($CDCl_3$) δ 7.56 (d,1H), 7.10-7.35 (m,6H), 6.26 (dd,1H), 4.30 (m,2H), 3.34 (m,1H), 3.12 (dd,1H), 2.72 (dd,1H).

Example 114

(2RS)-2-Benzyl-3-(N-methoxyl-N-methylamino)propionic Acid.

Using the procedure of Example 113 with the resultant compound from Example 111 gave the desired product. $^1H$ NMR ($CDCl_3$) δ 7.10-7.35 (m,5H), 3.58 (s,3H), 2.62 (s,3H).

Example 115

Methyl (2RS)-2-benzyl-3-tert-butylmercaptopropionate.

To sodium (3.05 g, 133 mmol) in methanol (135 ml) was added tert-butylmercaptan (17.0 ml, 151 mmol). After 20 min methyl α-benzylacrylate (17.05 g, 96.8 mmol) in methanol (100 ml) was added and after 1 h at room temperature the mixture was heated at reflux for 17 h. After cooling, the mixture was acidified with 2 M HCl (70 ml), concentrated, taken up in ether, washed with water and brine, then dried over $MgSO_4$ and evaporated to 23.59 g (92%) of an oil. $^1H$ NMR ($CDCl_3$) δ 7.15-7.35 (m,5H), 3.63 (s,3H), 2.60-3.05 (m,5H), 1.28 (s,9H).

Example 116

Methyl (2RS)-2-benzyl-3-tert-butylsulfonylpropionate.

To the resultant compound from Example 115 (270 mg, 1.01 mmol) in methanol (6 ml) and water (5 ml) at 0 °C was added potassium peroxymonosulfate (1.845 g, 6 mmol) in portions. After 15 min at 0 °C and 24 h at room temperature the mixture was filtered, diluted with water, and extracted with $CH_2Cl_2$ which was washed with brine, dried over $MgSO_2$, and evaporated to 300 mg (99%) of an oil. $^1H$ NMR ($CDCl_3$) δ 7.15-7.35 (m,5H), 3.68 (s,3H), 3.45 (m,2H), 3.12 (dd,1H), 2.98 (m,2H), 1.37 (s,9H).

Example 117

(2RS)-2-Benzyl-3-tert-butylsulfonylpropionic Acid.

The resultant compound from Example 116 (282 mg, 0.95 mmol) in 6 M HCl (2 ml) and acetic acid (0.4 ml) was heated at reflux for 16 h. The mixture was cooled and filtered and the resulting solid was recrystallized from methycyclohexane/ethyl acetate to afford 152 mg (56%) of the desired product, m.p. 147-148 °C.

Example 118

(2R,4R,5S)-2-(4-Pentenyl)-4-hydroxy-5-tert-butyloxycarbonylamino-6-phenylhexanoic Acid Amide of (2'S,1'R,5S)-3-Ethyl-5-(1′-hydroxy-2′-amino-3′-cyclohexylpropyl)oxazolidin-2-one.

Using the procedure of Evans, et al . (J. Org. Chem. 1985, 50 , 4615) with the resultant compound of Example 9 and (3 R ,5 R ,1′S)-5-(1-( t -butyloxycarbonylamino)-2-phenylethyl)-3-(4-pentenyl)dihydrofuran-2-(3 H )-one (D.J. Kempf, J. Org. Chem. 1986, 51 , 3921) gave the desired compound.

Example 119

tert-Butyl (2(S)-2-(tert-butyloxycarbamoyl)-3-phenylpropyl)sulfide.

tert-Butyl mercaptan (0.52 ml, 416 mg, 4.61 mmol) was added to a suspension of sodium hydride (111 mg, 4.62 mmol) in 8 ml of THF, cooled to 0 °C under a nitrogen atmosphere. The resulting suspension was stirred at 0 °C for 1 h, and for an additional 3 h at room temperature. To the resulting thick white suspension, cooled to 0 °C, was added dropwise via cannula, a solution of 1-phenyl-3-trifluorosulfonyloxy-2-t-butyloxyamidopropane (1.63 g, 4.02 mmol) in 10 ml of THF. The resultant mixture was allowed to stir and slowly warm to room temperature over 18 h. The mixture was partitioned between 75 ml $Et_2O$ and 50 ml water. The organic phase was extracted with 50 ml saturated $NaHCO_3$, then the combined aqueous phases were extracted with 2 x 50 ml $Et_2O$. All organic phases were combined, washed with 50 ml brine, dried ($MgSO_4$), and the filtrate concentrated under reduced pressured to afford 1.40 g of orangish solid. Purification by recrystallization (hexanes, three crops) gave 1.16 g (89%) of white crystals; m.p. 67-69 C. $^1H$ NMR ($CDCl_3$) $\delta$ 1.31 (s,9H), 1.43 (s,9H), 2.57 (dd,1H), 2.66 (dd,1H), 2.84 (m,2H), 4.04 (bm,1H), 4.79 (bm,1H), 7.16-7.4 (m,5H).

Example 120

tert-Butyl (2(S)-2-(tert-butyloxycarbamoyl)-3-phenylpropyl)sulfone.

The resultant compound from Example 119 (863 mg, 2.67 mmol) was dissolved in 5 ml absolute ethanol and 5 ml THF, and 2.5 ml water and 5 ml pH 4.5 aqueous phosphate buffer was added. The mixture was cooled in ice and treated with OXONE (2.45 g, 8.00 mmol $KHSO_5$). The mixture was stirred and allowed to warm to room temperature. After 60 h, the mixture was partitioned between 50 ml water and 50 ml $CH_2Cl_2$. The aqueous phase was further extracted with 3 x 50 ml $CH_2Cl_2$, and the combined organic extracts were washed with brine, dried ($MgSO_4$), filtered, and the filtrate concentrated under reduced pressure to give 942 mg of white solid. Recrystallization from $CH_2Cl_2/Et_2O$ (three crops) afforded 839 mg (88%) of white crystals, m.p. 169-170.5 °C. $^1H$ NMR ($CDCl_3$) $\delta$ 1.38 (s,9H), 1.42 (s,9H), 3.1-3.3 (m,4H), 4.30 (bm,1H), 5.26 (bs,1H), 7.21-7.42 (m,5H). High resolution mass spectrum; calcd. for $C_{13}H_{30}NO_4S$ $(M+H)^+$: 356.1895. Found: 356.1894.

Example 121

tert-Butyl (2(S)-2-amino-3-phenylpropyl) sulfone hydrochloride

The resultant compound from Example 120 (741 mg, 2.09 mmol) was treated with 2.5 ml of 4.5 M HCl in dioxane at room temperature. After 24 h the volatiles were removed under reduced pressure, and the residue placed under high vacuum overnight, to afford 614 mg (100%) of the desired compound; m.p. >220

°C. $^1H$ NMR ($CDCl_3$) δ 1.34 (s,9H), 3.20 (dd,1H), 3.26 (dd,1H), 3.70 (dd,1H), 3.89 (dd,1H), 4.25 (bm,1H), 7.25-7.35 (m,5H), 8.7 (bs,1H + $H_2O$).

## Example 122

### Diethyl 4-Thiazoylmethyl malonate.

Sodium metal (4.14 g, 180 mmol) was added to 150 ml of absolute ethanol and cooled in an ice-water bath under a nitrogen atmosphere. After the sodium dissolved diethyl malonate (28.83 g, 180 mmol) was added and the solution warmed to room temperature and stirred for 2 h. The reaction mixture was recooled in an ice-water bath and 4-chloromethylthiazole (2.4 g, 18.0 mmol) in 5 ml of ethanol was added dropwise over 10 min and stirred for 20 h at room temperature. The crude reaction mixture was concentrated then partitioned between 50 ml $H_2O$ and 50 ml $CH_2Cl_2$ the organic layer was filtered through $MgSO_4$ and concentrated. The crude product was chromatographed on silica gel with 10% EtOAc/hex to give 3.1 g (67%) of a clear oil. $^1H$ NMR ($CDCl_3$, TMS) δ (t,6H), 3.4 (d,2H), 3.9 (t,1H), 4.15 (gd,4H), 7.0 (d,1H), 8.7 (d,1H); IR ($CDCl_3$) 2900, 2250, 1740, 1290, 1050 $cm^{-1}$. Mass spectrum: $(M+H)^+$ = 258.
Anal. Calcd for $C_{11}H_{15}NO_4S$: C, 51.34; H, 5.88; N, 5.44.
Found: C, 50.84; H, 5.82; N, 5.39.

## Example 123

### Ethyl 4-Aza-5(S)-benzyl-6-t-burylsulfonyl-3-oxo-2(R,S)-(4-thiazoylmethyl)hexanoate

To a solution. of 0.5 g (1.9 mmol) of the diethyl malonate (Example 122) in 25 ml of absolute ethanol cooled in an ice-water bath and stirred under a nitrogen atmosphere was added a solution of potassium hydroxide (0.11 g, 1.9 mmol) in 25 ml of absolute ethanol. Upon complete addition, the reaction was stirred for 72 h at room temperature and concentrated to afford the crude acid salt. The acid salt was redissolved into 30 ml of DMF, cooled in an ice-water bath. 1-Hydroxybenzotriazole (HOBT) (0.38 g, 2.88 mmol), 4-methyl morpholine (0.28 g, 2.8 mmol), tert-butyl-(2(S)-2-amino-3-phenylpropyl)sulfone hydrochloride (0.55 g, 1.9 mmol), and (1-(3-dimethylaminopropyl)-3-ethylcarbodiimide hydrochloride (EDAC) (0.36 g, 1.9 mmol) were added. After 18 h, the solution was diluted with 50 ml saturated aqueous $NaHCO_3$ and extracted with 30 ml EtOAc (3X). The combined ethyl acetate extracts were washed with 25 ml 0.5 N HCl (2X), filtered through $MgSO_4$, and taken to dryness. The crude product was chromatographed on silica gel with 1:1 EtOAc/hex to give 0.78 g (88%) of a white solid. $^1H$ NMR ($CDCl_3$, TMS) δ 1.2 (td,6H), 1.4 (d,9H), 1.7 (s,2H-($H_2O$)), 3.0-3.3 (m,4H), 3.35 (dd,2H), 3.74 (2dd,1H), 4.12 (m,4H), 4.54 (m,1H), 7.0 (dd,1H), &.1 (bd,1H), 7.25 (m,5H), 8.65 (dd,1H); IR ($CDCl_3$) 3420, 2900, 2250, 1740, 1670, 1520, 1290, 1140 $cm^{-1}$. Mass spectrum: $(M+H)^+$ = 467.
Anal. Calcd. for $C_{22}H_{30}N_2O_5S_2$: C, 56.63; H, 6.48; N, 6.01.
Found: C, 56.79; H, 6.56; N, 6.01.

## Example 124

### 3(S)-Acetoxy-2(R,S)-benzyl-3-morpholinocarbonylpropionic Acid

To a solution of 0.55 g (1.9 mmol) of diethyl (2S,3R,S)-3-benzyl-2-hydroxysuccinate, prepared according to the procedure of D. Seebach, Org. Syn, 63, 109, in 8 ml of tetrahydrofuran, cooled in an ice-water

bath was added 220 mg (5.2 mmol) of lithium hydroxide mono hydrate in 8 ml of water. The bath was removed and the reaction stirred for 18 h. The reaction mixture was acidified with concentrated HCl until pH 4 and the solvents removed under high vacuum to give a white solid. The crude diacid was warmed to 50 °C in 4 ml of 1:1 acetic anhydride:acetyl chloride for 3 h. The excess acetic anhydride-acetyl chloride was removed under high vacuum. The residue was dissolved in dichloromethane, cooled in an ice-water bath, and 2 ml of morpholine was added. The reaction was stirred for 1 h, diluted with 1 N HCl, and the crude product extracted with chloroform. The combined chloroform extracts were dried ($MgSO_4$), filtered, and concentrated to give a brown oil. The product was purified by silica gel chromatography using 5:95:0.02 methanol:dichloromethane:acetic acid as eluant. Mass spectrum: $(M+H)^+$ = 336.

Example 125

(2R)-2-Benzyl-3-((2-diethylaminoethyl)methylaminocarbonyl)propionic acid amide of L-(4-thiazolyl)alanine-2(S)-amino-1-cytclohexyl-4,4-difluoro-3(R),5(R)-dihydroxy-6-methylheptane

The protecting group of the resultant compound from Example 68 was removed using 4 M HCl/dioxane. The resulting amine hydrochloride was coupled to the acid from Example 107 using the procedure of Example 14.

Example 126

(2R)-2-Benzyl-3-thiazol-4-ylpropionic acid amide of L-(4-thiazolyl)alanine-2(S)-amino-1-cyclohexyl-4,4-difluoro-3-(R),5(R)-dihydroxy-6-methylheptane

The protecting group of the resultant compound from Example 68 was removed using 4 M HCl/dioxane. The resulting amine hydrochloride was coupled to the acid from Example 109 using the procedure of Example 14.

Example 127

(2R)-2-Benzyl-3-pyrazol-1-ylpropionic acid amide of L-(4-thiazolyl)alanine-2(S)-amino-1-cyclohexyl-4,4-difluoro-3-(R),5(R)-dihydroxy-6-methylheptane

The protecting group of the resultant compound from Example 68 was removed using 4 M HCl/dioxane. The resulting amine hydrochloride was coupled to the acid from Example 113 using the procedure of Example 14. The desired diastereomer was isolated by chromatography.

Example 128

(2R)-2-Benzyl-3-(N-methoxyl-N-methylamino)propionic acid amide of L-(4-thiazolyl)alanine-2(S)-amino-1-cyclohexyl-4,4-difluoro-3(R),5(R)-dihydroxy-6-methylheptane

The protecting group of the resultant compound from Example 68 was removed using 4 **M** HCl/dioxane. The resulting amine hydrochloride was coupled to the acid from Example 114 using the procedure of Example 14. The desired diastereomer was isolated by chromatography.

## Example 129

(2S)-2-Benzyl-3-tert-butylsulfonylpropionic acid amide of L-(4-thiazolyl)alanine-2(S)-amino-1-cyclohexyl-4,4-difluoro-3(R),5(R)-dihydroxy-6-methylheptane

The protecting group of the resultant compound from Example 68 was removed using 4 **M** HCl/dioxane. The resulting amine hydrochloride was coupled to the acid from Example 117 using the procedure of Example 14. The desired diastereomer was isolated by chromatography.

## Example 130

4-Aza-5(S)-benzyl-6-t-butylsulfonyl-3-oxo-2(R,S)-(4-thiazolvmethyl)hexane carboxamide of L-(4-thiazolyl)alanine-2-(S)-amino-1-cyclohexyl-4,4-difluoro-3(R),5(R)-dihydroxy-6-methylheptane

The ethyl ester (0.20 g, 0.4 mmol) obtained from Example 123 and potassium hydroxide (0.024 g, 0.4 mmol) in 2 ml of absolute ethanol were stirred at room temperature under nitrogen atmosphere for 18 h and concentrated. The resulting acid salt and the deprotected product from Example 68 were coupled according to the procedure of Example 14. The desired diastereomer was isolated by chromatography.

## Example 131

3(S)-Acetoxy-2(R)-benzyl-3-morpholinocarbonylpropionic acid amide of L-(4-thiazolyl)alanine-2(S)-amino-1-cyclohexyl-4,4-difluoro-3(R),5(R)-dihydroxy-6-methylheptane

The protecting group of the resultant compound from Example 68 was removed using 4 **M** HCl/dioxane. The resulting amine hydrochloride was coupled to the acid from Example 124 using the procedure of Example 14. The desired diastereomer was isolated by chromatography.

## Example 132

3(S)-Hydroxy-2(R)-benzyl-3-morpholinocarbonylpropionic acid amide of L-(4-thiazolyl)alanine-2(S)-amino-1-cyclohexyl-4,4-difluoro-3(R),5(R)-dihydroxy-6-methylheptane

Using the procedure of Example 113 with the resultant compound from Example 131 gave the desired product.

## Example 133

(2R)-2-Benzyl-3-(N-Methyl-N-2-methoxyethoxymethoxyethylaminocarbonyl) propionic acid amide of L-(4-thiazolyl)alanine-2(S)-amino-1-cyclohexyl-4,4-difluoro-3(R),5(R)-dihydroxy-6-methylheptane

The protecting group of the resultant compound from Example 68 was removed using 4 **M** HCl/dioxane. The resulting amine hydrochloride was coupled to (2R)-2-benzyl-3-(N-Methyl-N-2-methoxyethoxymethoxyethylaminocarbonyl) propionic acid (Rosenberg et al. *J. Med. Chem.* **1990**, *33*, 1962) using the procedure of Example 14.

Example 134

(2R)-2-Benzyl-3-(N-Methyl-N-2-methoxyethylaminocarbonyl) propionic acid amide of L-(4-thiazolyl)alanine-2(S)-amino-1-cyclohexyl-4,4-difluoro-3(R),5(R)-dihydroxy-6-methylheptane

The protecting group of the resultant compound from Example 68 was removed using 4 **M** HCl/dioxane. The resulting amine hydrochloride was coupled to (2R)-2-benzyl-3-(N-Methyl-N-2-methoxyethylaminocarbonyl) propionic acid (Rosenberg et al. *J. Med. Chem.* **1990**, *33*, 1962) using the procedure of Example 14.

Example 135

Dibenzyl acetic acid amide of L-(4-thiazolyl)alanine-2(S)-amino-1-cyclohexyl-4,4-difluoro-3(R),5(R)-dihydroxy-6-methylheptane.

The protecting group of the resultant compound from Example 68 was removed using 4 **M** HCl/dioxane. The resulting amine hydrochloride was coupled to dibenzyl acetic acid using the procedure of Example 14.

Example 136

Methyl 3-Hydroxy-2-methylene-3-phenylpropionate

A mixture of benzaldehyde (82.1 mL, 0.81 mol), methyl acrylate (109.1 mL, 1.211 mol), 1,4-diazabicyclo(2,2,2) octane (13.6 g, 0.12 mol), and acetic acid (1.4 mL, 0.024 mol) was allowed to stir at 35 °C for 60 h, at which point the reaction was determined to have proceeded to 70% completion by $^1$H NMR. Methyl acrylate (20.9 mL, 0.23 mol) was then added and the solution was allows to react at 35 °C for an additional 48 h. The mixture was diluted with diethyl ether (1.0 L) and was washed with 2 × 200 mL portions of a pH 7 phosphate buffer. After concentration in vacuo, the remaining mixture was distilled at reduced pressure (12 mm) to afford 6.5 g of unreacted benzaldehyde and 130.0 g (90%) of the desired product as a colorless oil: b.p. 130 °C (12 mm); IR (film) 1718, 1440 cm$^{-1}$; $^1$H NMR ($CDCl_3$) δ 3.67 (s, 3H), 5.52 (br s, 1H), 5.83-5.85 (m, 1H), 6.29-6.31 (m, 1H), 7.23-7.39 (m, 5)H; $^{13}$C NMR (75 MHz, $CDCl_3$) δ 51.8, 72.9, 125.8, 126.5, 127.7, 128.3, 141.2, 141.9, 166.6.

Example 137

(Z)-1-Bromo-2-carbomethoxy-3-phenyl-2-propene

To a 2 L, 3-neck Morton flask fitted with a thermometer, a mechanical stirrer, and an addition funnel was added the resultant compound from Example 136 (305.9 g, 1.585 mol) followed by addition of 48% HBr (505 mL, 4.46 mol) in one portion. The flask was immersed in an ice-water bath, at which time concentrated sulfuric acid (460 mL, 8.62 mol) was added dropwise over 90 min and the internal temperature of the reaction mixture was maintained at 23-27 °C throughout the addition process. After removal of the ice-water bath, the mixture was allowed to stir at ambient temperature overnight. The solution was then transferred to a separatory funnel and the organic layer was allowed to separate from the acid layer. The acids were drained and the organic layer was diluted with 2 L of a 1:1 ethyl acetate/hexane solution, washed with saturated aqueous sodium bicarbonate solution (1 L), dried over sodium sulfate, and concentrated to yield 400 g (99%) of the desired product as a light yellow oil, which was used without any additional purification: b.p. 180 °C (12 mm); IR (film) 1718, 712 $cm^{-1}$; $^{1}H$ NMR ($CDCl_3$) $\delta$ 3.89 (s, 3H), 4,40 (s, 2H), 7.38-7.45 (m, 3H), 7.56-7.60 (m, 2H), 7.83 (s, 1H); $^{13}C$ NMR (75 MHz, $CDCl_3$) $\delta$ 26.77, 52.47, 128.63, 128.87, 129.61, 134.20, 142.95, 166.62.

Example 138

(Z)-2-Carbomethoxy-3-phenyl-2-propene-1-sulfonic Acid Sodium Salt

To a 12 L, 3-neck round bottom flask fitted with a mechanical stirrer, thermometer and an addition funnel was added the resultant product from Example 137 (400 g, 1.57 mol) and methanol (4 L). The mixture was warmed to 50 °C and a solution of sodium sulfite (199 g, 1.57 mol) dissolved in water (4 L) was added over 75 min while the internal temperature of the flask was maintained at 50 °C. After the addition was complete, the clear solution was allowed to stir at 50 °C for an additional 45 min. The reaction mixture in solution was taken to the next step without additional purification. The compound may be isolated by concentration to an amorphous powder, which is contaminated with an equivalent of sodium bromide: IR (KBr) 1711, 1628, 1215 $cm^{-1}$; $^{1}H$ NMR (DMSO D-6) $\delta$ 3.70 (s, 3H), 3.77 (s, 2H), 7.33-7.41 (m, 3H), 7.48 (s, 1H), 7.87-7.89 (m, 2H); $^{13}C$ NMR (75 MHz, DMSO D-6) $\delta$ 49.88, 51.93, 127.36, 128.33. 128.91, 129.82, 134.75, 139.06, 168.60.

Example 139

2-Carbomethoxy-3-phenylpropane-1-sulfonic Acid Sodium Salt

To the 8 L of 1:1 methanol/water mixture containing the resultant compound from Example 138 was added 60 g of W-24 raney nickel. The resulting suspension was pressurized under 50 psi of hydrogen and was allowed to shake on a Parr shaker for 24 h, at which time an additional 20 g of raney nickel catalyst was added. After 6 h under 50 psi of hydrogen, the catalyst was removed by filtration and the solution was concentrated to dryness. To the dry white solid was added ethyl acetate (6 L) and heptane (4 L) and the solution was vigorously stirred with a mechanical stirrer overnight. The white suspension was removed by filtration yielding 530 g (88%) of the desired product as an amorphous powder that was contaminated with approximately one equivalent of NaBr. The compound was used without any additional purification: IR (KBr) 1740, 1215, 1050 $cm^{-1}$. $^{1}H$ NMR (DMSO D-6) $\delta$ 2.48-2.54 (m, 1H), 2.74-2.87 (m, 2H), 2.91-3.04 (m, 2H), 3.48 (s, 3H), 7.12-7.32 (m, 5H); $^{13}C$ NMR (75 MHz, $D_2O$/DMSO D-6) $\delta$ 38.18, 44.80, 52.67, 52.82, 127.42, 129.13, 129.34, 138.14, 176.84.

EXAMPLE 140

2-Carbomethoxy-3-phenyl-1-propanesulfonyl Chloride

To a 3 L round bottom flask was added the resultant compound from Example 139 (530 g, 1.39 mol) and toluene (520 mL) followed by the addition of $PCl_5$ (317 g, 1.52 mol). The mixture was warmed to 50 °C with stirring for 45 min. It was then diluted with toluene (1 L) and was filtered through celite. After concentration in vacuo, 371 g (96%) of the desired product was obtained as a light brown oil: IR (film); 1740, 1380, 1170 $cm^{-1}$; $^{1}H$ NMR ($CDCl_3$); δ 2.92 (dd, 1H, J = 8.1, 14.0), 3.17 (dd, 1H, J = 6.6, 14.0), 3.41-3.50 (m, 1H), 3.67 (dd, 1H, J = 3.3, 14.3), 3.72 (s, 3H), 4.20 (dd, 1H, J = 8.8, 14.3), 7.15-7.18 (m, 2H), 7.25-7.35 (m, 3H); $^{13}C$ NMR (75 MHz, $CDCl_3$) δ 37.26, 42.88, 52.65, 64.89, 127.49, 128.87, 128.92, 135.61, 171.79.

EXAMPLE 141

Methyl 2-Benzyl-3-(1-methyl-piperidin-4-ylsulfonyl)propionate

To a 1 L round bottom flask was added the resultant compound from Example 140 (84.5 g, 0.305 mol) and dichloromethane (305 mL). The mixture was cooled to 0 °C in an ice water bath and a solution of N-methyl piperazine (35.5 mL, 32.1 g) dissolved in dichloromethane (305 mL) was added dropwise with vigorous stirring over 90 min. After the addition was completed, the ice-water bath was removed and the mixture was stirred an additional 4 h while warming to ambient temperature. The solution was then poured into a separatory funnel containing 1 L of a 5% aqueous NaOH solution. The layers were partitioned and the organic layer was dried over potassium carbonate. Concentration in vacuo yielded an oil, which was filtered through 200 g of silica gel using 4:1 hexane/ethyl acetate as an eluant. Concentration gave 84.3 g (81%) of the desired product as a yellow oil: IR (film); 1735, 1165, 955 $cm^{-1}$; $^{1}H$ NMR ($CDCl_3$) δ 2.30 (s, 3H), 2.42 (t, 4H, J = 4.8), 2.88 (dd, 1H, J = 7.7, 14.0), 2.93. (dd, 1H, J = 3.7, 14.0), 3.06 (dd, 1H, J = 7.0, 13.6), 3.18-3.27 (m, 5H), 3.43 (dd, 1H, J = 8.82, 13.9), 3.67 (s, 3H), 7.14-7.17 (m, 2H), 7.24-7.34 (m, 3H); $^{13}C$ NMR (75 MHz, $CDCl_3$) δ 37.91, 42.22, 45.36, 45.83, 49.61, 52.21, 54.36, 127.06, 128.66, 128.92, 129.06, 136.79, 173.33.

Example 142

(2S) 2-Benzyl-3-(1-methyl-piperidin-4-ylsulfonyl)propionic Acid

The resultant racemic ester from Example 141 (135 g, 397 mmol) was suspended in acetone (300 mL) and water (900 mL). While being stirred vigorously at a temperature of 35 °C, a crude preparation of Subtilisin Carlsberg (10 mL, Alcalase 2.4L, Novo Laboratories) was added. Sodium hydroxide solution (6 **M**) was used to maintain the reaction at pH 7.5-8.0. After 3 days, the acetone was removed under reduced pressure and the aqueous phase was extracted with $CHCl_3$ (1 L) to remove the unreacted ester. The aqueous phase was adjusted to pH 7 with 3 **M** HCl and was desalted by eluting through a column of Amberlite XAD-16(2 kg, prewashed sequentially with water, methanol, and water) using a water to water/methanol gradient. Evaporation of the solvent afforded 46 g (70%) of a white solid: mp 184.5 °C; TLC (25% ethyl acetate/25% water/25% acetic acid/25% n-butanol) $R_f$ = 0.43;
Anal.($C_{15}H_{22}N_2O_4S \cdot 0.25\ H_2O$)
Calcd: C, 54.44; H, 6.85; N, 8.47.
Found: C, 54.77; H, 6.53; N, 8.39.

Example 143

(2S) 2-Benzyl-3-(1-methyl-piperidin-4-ylsulfonyl)propionic acid amide of L-(4-thiazolyl)alanine-2(S)-amino-1-cyclohexyl-4,4-difluoro-3(R),5(R)-dihydroxy-6-methylheptane

The protecting group of the resultant compound from Example 68 was removed using 4 **M** HCl/dioxane. The resulting amine hydrochloride was coupled to the acid from Example 142 using the procedure of Example 14.

Example 144

Benzyl (2R)-2-Benzyl-3-[(4-cyclopropylpiperazin-1-yl)carbonyl]propionate

Using the procedure of Example 62 and replacing 1-trifluoroethylpiperazine with 1-cyclopropylpiperazine gave the desired product. $^1H$ NMR ($CDCl_3$) δ 7.10-7.34 (5H,m), 5.11 (2H,dd), 3.5l (2H,m), 3.31 (3H,m), 3.04 (1H,dd), 2.74 (2H,m), 2.52 (3H,m), 2.35 (1H,dd), 0.47 (2H,m), 0.42 (2H,m).

Example 145

(2R)-2-Benzyl-3-[(4-cyclopropylpiperazin-1-yl)carbonyl]propionic Acid

Using the procedure of Example 63 with the resultant compound from Example 144 gave the desired product.

Example 146

Benzyl (2R)-2-Benzyl-3-[(N-pyridin-4-yl)methylaminocarbonyl]propionate.

Using the procedure of Example 62 but replacing 1-trifluoroethylpiperazine with 4-methylaminopyridine provided the desired product. $^1H$ NMR ($CDCl_3$) δ 8.6 (m,2H), 7.4-7.0 (m,12H), 5.1 (q,2H), 3.3 (m,1H), 3.2 (s,3H), 3.0 (dd,1H), 2.7 (dd,1H), 2.6 (dd,1H), 2.25 (dd,1H).

Example 147

(2R)-2-Benzyl-3-[(N-pyridin-4-yl)methylaminocarbonyl]propionic Acid

Prepared from the resultant compound of Example 146 according to the procedure of Example 63, m.p. 88-92 °C.

Example 148

1-Benzyloxycarbonyl-3-hydroxyazetidine.

1-Diphenylmethyl-4-hydroxyazetidine (1.00 g, 4.18 mmol) and 10% Pd/C in methanol (10 mL) were stirred under a hydrogen atmosphere for 20 h. The mixture was filtered and evaporated, and the residue was dissolved in methylene chloride and cooled to 0° C. After addition of triethylamine (0.64 mL, 4.57 mmol) and benzyl chloroformate (0.60 mL, 4.20 mmol), the mixture was stirred at room temperature for 90 min. The mixture was evaporated, taken up in ethyl acetate, washed with 2 M HCl, saturated $NaHCO_3$ solution and brine, and then dried over $Na_2SO_4$ and evaporated. Chromatography of the residue on silica gel (120 g) with 50-60% ethyl acetate in hexane afforded 0.376 g (43%) of a colorless oil. TLC (50% ethyl acetate/50% hexane) $R_f$ = 0.13; $^1H$ NMR ($CDCl_3$) δ 7.29-7.39 (5H,m), 5.10 (2H,s), 4.59-4.70 (1H,m), 4.26 (1H,dd), 4.23 (1H,dd), 3.91 (1H,dd), 3.88 (1H,dd), 2.15 (1H,d).

Example 149

3-Acetylmercapto-1-benzyloxycarbonylazetidine.

To triphenylphosphine (4,40 g, 16.8 mmol) in tetrahydrofuran (25 mL, THF) at -78° C was added diethylazodicarboxylate (2.60 mL, 16.5 mmol) in THF (15 mL). After 7 min thiolacetic acid (1.25 mL, 17.5 mmol) in THF (15 mL) was added followed by, after 7 min, the resultant compound from Example 148 (2.789 g, 13.46 mmol). The mixture was stirred at -78° C for 1 h and then at room temperature for 20 h, and was then evaporated and chromatographed on silica gel (300 g) with 20% ethyl acetate in hexane affording 3.250 g (91%) of a white solid, m.p. 94.5-95.5° C. TLC (20% ethyl acetate/80% hexane) $R_f$ = 0.17; $^1H$ NMR ($CDCl_3$) δ 7.28-7.41 (5H,m), 5.09 (2H,s), 4,48 (1H,d), 4,44 (1H,d), 4.15-4.26 (1H,m), 3.92 (1H,d), 3.89 (1H,d) 2.34 (3H,s).
Anal ($C_{13}H_{15}NO_3S$).
Calcd: C, 58.85; H, 5.70, N, 5.28.
Found: C, 58.81; H, 5.70; N, 5.26.

Example 150

Methyl 2-Benzyl-3-(1-benzyloxycarbonylazetidin-3-ylmercapto)propionate.

A solution of sodium methoxide in methanol (3mL) prepared with sodium bis(trimethylsilyl)amide (0.75 mL, 0.75 mmol, 1.0 M in THF) was added to the resultant compound from Example 149 (205.0 mg, 0.773 mmol) in methanol (3 mL). After 45 min, methyl α-benzylacrylate (150.0 mg, 0.851 mmol) in methanol (2 mL) was added. After 45 min the reaction was quenched with 2 M HCl (0.38 mL, 0.76 mmol), evaporated, chromatographed on silica gel (30 g) with 20% ethyl acetate in hexane, to afford 280.6 mg (91%) of a colorless oil. TLC (20% ethyl acetate/80% hexane) $R_f$ = 0.13; $^1H$ NMR ($CDCl_3$) δ 7.10-7.40 (10H,m), 5.08 (2H,s), 4.21-4.33 (2H,m), 3.77-3.90 (2H,m), 3.66 (3H,s), 3.53-3.63 (1H,m), 3.00 (1H,dd), 2.72-2.90 (3H,m), 2.63 (1H,dd).

Example 151

Methyl 2-Benzyl-3-(1-benzyloxycarbonylazetidin-3-ylsulfonyl)propionate.

The resultant compound from Example 150 (276.0 mg, 0.691 mmol) in methanol (6 mL) and water (5 mL) was treated with OXONE (1.27 g, 2.07 mmol). After 14 h the mixture was diluted with methanol, filtered and concentrated to ca. 5 mL. After neutralization with solid $K_2CO_3$ the mixture was extracted into ethyl acetate which was washed with saturated $NaHCO_3$ solution, water, and brine, and then was dried over $Na_2SO_4$ and evaporated to afford 295.9 mg (99%) of a colorless oil. TLC (50% ethyl acetate/50% hexane) $R_f$ = 0.18; $^1H$ NMR ($CDCl_3$) $\delta$ 7.10-7.40 (10H,m), 5.09 (2H,s), 4.22-4.35 (2H,m), 4.25 (1H,dd), 4.12 (1H,dd), 3.80-3.92 (1H,m), 3.73 (3H,s), 3.44 (1H,dd), 3.27-3.38 (1H,m), 3.14 (1H,dd), 2.92 (1H,dd), 2.87 (1H,dd).

Example 152

Methyl 2-Benzyl-3-(1-methylazetidin-3-ylsulfonyl)propionate.

The resultant compound from Example 151 (270.8 mg) and 10% Pd/C (150 mg) in methanol (6 mL) was treated with formaldehyde in water (0.25 mL, 37% formalin) and stirred under a hydrogen atmosphere for 3 h. The mixture was filtered and evaporated to afford 194.3 mg (99%) of a colorless oil. TLC (15% $CH_3OH$/85% $CHCl_3$) $R_f$ = 0.60; $^1H$ NMR ($CDCl_3$) $\delta$ 7.12-7.37 (5H,m), 3.77 (1H,dd), 3.71 (3H,s), 3.56 (1H,dd), 3.38-3.50 (4H,m), 3.26-3.36 (1H,m), 3.12 (1H,dd), 2.96 (1H,dd), 2.88 (1H,dd), 2.32 (3H,s).

Example 153

2-Benzyl-3-(1-methylazetidin-3-ylsulfonyl)propionic Acid Hydrochloride.

The resultant compound from Example 152 (2.120 g, 6.81 mmol) in 2 M HCl was stirred at 75° C for 20 h. The mixture was washed with ether, evaporated with water chasers, and lyophillized to afford 2.075 g (91%) of a white foam. TLC (25% ethyl acetate/25% water/25% acetic acid/25% n-butanol) $R_f$ = 0.50; $^1H$ NMR ($CD_3OD$) $\delta$ 7.17-7.35 (5H,m), 3.58-3.68 (2H,m), 2.95 (3H,s).

Example 154

Benzyl (2R)-2-Benzyl-3-(2-dimethylaminothiazol-4-yl)propionate.

The resultant compound from Example 69 (182.0 mg, 0.55 mmol) and N,N-dimethylthicurea (86 mg, 0.83 mmol) in acetone (4 ml) were stirred at room temperature for 48 h. The mixture was evaporated, taken up in ethyl acetate, washed with saturated $NaHCO_3$ solution and brine, then dried over $Na_2SO_4$ and evaporated. Chromatography on silica gel (18 g) with 20% ethyl acetate in hexane afforded 194 mg (93%) of an oil. TLC (50% ethyl acetate/50% hexane) $R_f$ = 0.66; $^1H$ NMR ($CDCl_3$) $\delta$ 7.10-7.30 (m,10H), 6.06 (s,1H), 5.01 (d,1H), 4.97 (d,1H), 3.15-3.30 (m,2H), 3.04 (s,6H), 2.88-3.00 (m,1H), 2.87 (dd,1H), 2.77 (dd,1H).

Example 155

(2R)-2-Benzyl-3-(2-dimethylaminothiazol-4-yl)propionic Acid.

Using the procedure of Example 109 with the resultant compound from Example 154 gave the desired product as an oil. TLC (10% $CH_3OH$/90% $CHCl_3$) $R_f$ = 0.47; $^1H$ NMR ($CDCl_3$) δ 7.15-7.35 (m,5H), 5.95 (s,1H), 3.25 (dd,1H), 3.12 (s,6H), 3.00-3.15 (m,1H), 2.60-2.90 (m,3H).

Example 156

Benzyl (2R)-2-Benzyl-3-(2-methylimino-3-methyl-2,3-dihydrothiazol-4-yl)propionate.

The resultant compound from Example 69 (355.0 mg, 1.07 mmol) and N,N′-dimethylthiourea (98 mg, 0.94 mmol) in acetone (6 ml) were stirred at room temperature for 162 h. The mixture was evaporated, taken up in ethyl acetate, washed with 1.0 M $Na_2CO_3$ solution and brine, then dried over $Na_2SO_4$ and evaporated. Chromatography on silica gel (19 g) with 3% methanol in chloroform afforded 319 mg (89%) of an oil. TLC (10% $CH_3OH$/90% $CHCl_3$) $R_f$ = 0.40; $^1H$ NMR ($CDCl_3$) δ 7.10-7.35 (m,10H), 5.51 (s,1H), 5.05 (d,1H), 5.00 (d,1H), 3.12 (s,3H), 2.93-3.08 (m,2H), 2.97 (s,3H), 2.73-2.88 (m,2H), 2.51 (ddd,1H).

Example 157

(2R)-2-Benzyl-3-(2-methylimino-3-methyl-2,3-dihydrothiazol-4-yl)propionic Acid Hydrobromide.

The resultant compound from Example 156 (315 mg, 0.828 mmol) was stirred for 2 h in 30% HBr in acetic acid (5 ml). The solvent was evaporated and the residue was dissolved in water which was washed with ether and lyophillized to afford 310 mg (100%) of the desired product as a foam. $^1H$ NMR ($CD_3OD$) δ7.20-7.35 (m,5H), 6.72 (s,1H), 3.44 (s,3H), 3.08 (s,3H), 2.70-3.20 (m,5H).

Example 158

Benzyl (2R)-2-Benzyl-3-(5,6-dihydroimidazo[2,1-b]thiazol-3-yl)propionate.

Using the procedure of Example 156 but replacing N,N′-dimethylthiourea with 2-imidazolidinethione gave the desired product as an oil. TLC (10% $CH_3OH$/90% $CHCl_3$) $R_f$ = 0.32; $^1H$ NMR ($CD_3OD$) δ 7.15-7.35 (m,10H), 6.42 (s,1H), 5.06 (d,1H), 5.01 (d,1H), 4.15-4.32 (m,4H), 2.70-3.20 (m,4H), 2.77 (ddd,1H).

Example 159

(2R)-2-Benzyl-3-(5,6-dihydroimidazo[2,1-b]thiazol-3-yl)propionic Acid Hydrobromide.

Using the procedure of Example 157 with the resultant compound from Example 158 gave the desired product as a foam. TLC (25% ethyl acetate/25% water/25% acetic acid/25%n-butanol) $R_f$ = 0.51; $^1H$ NMR ($CD_3OD$) δ 7.20-7.35 (m,5H), 6.51 (s,1H), 4.32 (s,4H), 3.00-3.15 (m,2H), 2.83-2.95 (m,2H), 2.72 (ddd,1H).

Example 160

[2-Pyridin-2-ylethyl(methyl)aminolsulfonyl Chloride Hydrochloride.

2-(2-Methyaminoethyl)pyridine (10 mmol) was treated with excess HCl in ethanol. The mixture was evaporated and the residue was chased several times with ether and dried under high vacuum. The resulting dihydrochloride was treated with $SO_2Cl_2$•(30 mmol) in acetonitrile (15 mL). The mixture was heated at reflux for 24 h, cooled and filtered and the resulting solid was used with no further purification.

Example 161

[2-pyridin-2-ylethyl(methyl)aminolsulfonyl-(O-benzyl)threonine Methyl Ester.

To the resulting compound from Example 160 (1 mmol) and (O-benzyl)threonine methyl ester (1 mmol) in $CH_2Cl_2$ was added triethylamine (2.0 mmol). After 2 h the mixture was evaporated, suspended in ethyl acetate, washed with saturated $NaHCO_3$ solution, water and brine, and then dried over $Na_2SO_4$ and evaporated.

Example 162

[2-pyridin-2-ylethyl(methyl)amino]sulfonyl-(O-benzyl)threonine.

Using the procedure of Example 113 with the resultant compound of Example 161 gave the desired product.

Example 163

Boc-Met amide of 2(S)-amino-1-cyclohexyl-4,4-difluoro-3(R),5(R)-dihydroxy-6-methylheptane

Using the procedure of Example 14 but replacing Boc-His-OH with Boc-Met-OH gives the desired product.

Example 164

[2-pyridin-2-ylethyl(methyl)amino]sulfonyl-(O-benzyl)threonine-Methionine amide of 2(S)-amino-1-cyclohexyl-4,4-difluoro-3(R),5(R)-dihydroxy-6-methylheptane

The protecting group of the resultant compound from Example 163 was removed using 4 **M** HCl/dioxane. The resulting amine hydrochloride was coupled to the acid from Example 162 using the

procedure of Example 14.

Example 165

(2R)-2-Benzyl-3-[(4-cyclopropylpiperazin-1-yl)carbonyl]propionic acid amide of L-(4-thiazolyl)alanine-2(S)-amino-1-cyclohexyl-4,4-difluoro-3(R),5(R)-dihydroxy-6-methylheptane

The protecting group of the resultant compound from Example 68 was removed using 4 **M** HCl/dioxane. The resulting amine hydrochloride was coupled to the acid from Example 145 using the procedure of Example 14.

Example 166

(2R)-2-Benzyl-3-[(N-pyridin-4-yl)methylaminocarbonyl]propionic acid amide of L-(4-thiazolyl)alanine-2(S)-amino-1-cyclohexyl-4,4-difluoro-3(R),5(R)-dihydroxy-6-methylheptane

The protecting group of the resultant compound from Example 68 was removed using 4 **M** HCl/dioxane. The resulting amine hydrochloride was coupled to the acid from Example 147 using the procedure of Example 14.

Example 167

(2S)-2-Benzyl-3-(1-methylazetidin-3-ylsulfonyl)propionic acid amide of L-(4-thiazolyl)alanine-2(S)-amino-1-cyclohexyl-4,4-difluoro-3(R),5(R)-dihydroxy-6-methylheptane

The protecting group of the resultant compound from Example 68 was removed using 4 **M** HCl/dioxane. The resulting amine hydrochloride was coupled to the acid from Example 153 using the procedure of Example 14. The desired diastereomer was isolated by chromatography.

Example 168

(2R)-2-Benzyl-3-(2-dimethylaminothiazol-4-yl)propionic acid amide of L-(4-thiazolyl)alanine-2(S)-amino-1-cyclohexyl-4,4-difluoro-3(R),5(R)-dihydroxy-6-methylheptane

The protecting group of the resultant compound from Example 68 was removed using 4 **M** HCl/dioxane. The resulting amine hydrochloride was coupled to the acid from Example 155 using the procedure of Example 14.

Example 169

(2R)-2-Benzyl-3-(2-methylimino-3-methyl-2,3-dihydrothiazol-4-yl)propionic acid amide of L-(4-thiazolyl)alanine-2-(S)-amino-1-cyclohexyl-4,4-difluoro-3(R),5(R)-dihydroxy-6-methylheptane

The protecting group of the resultant compound from Example 68 was removed using 4 **M** HCl/dioxane. The resulting amine hydrochloride was coupled to the acid from Example 157 using the procedure of Example 14.

Example 170

(2R)-2-Benzyl-3-(5,6-dihydroimidazo[2,1-b]thiazol-3-yl)propionic acid amide of L-(4-thiazolyl]alanine-2(S)-amino-1-cyclohexyl-4,4-difluoro-3(R),5(R)-dihydroxy-6-methylheptane

The protecting group of the resultant compound from Example 68 was removed using 4 **M** HCl/dioxane. The resulting amine hydrochloride was coupled to the acid from Example 159 using the procedure of Example 14.

The compounds of the present invention can be used in the form of salts derived from inorganic or organic acids. These salts include but are not limited to the following: acetate, adipate, alginate, citrate, aspartate, benzoate, benzenesulfonate, bisulfate, butyrate, camphorate, camphorsulfonate, digluconate, cyclopentanepropionate, dodecylsulfate, ethanesulfonate, glucoheptonate, glycerophosphate, hemisulfate, heptonate, hexanoate, fumarate, hydrochloride, hydrobromide, hydroiodide, 2-hydroxy-ethanesulfonate, lactate, maleate, methanesulfonate, nicotinate, 2-naphthalenesulfonate, oxalate, pamoate, pectinate, persulfate, 3-phenylpropionate, picrate, pivalate, propionate, succinate, tartrate, thiocyanate, tosylate, and undecanoate. Also, the basic nitrogen-containing groups can be quaternized with such agents as loweralkyl halides, such as methyl, ethyl, propyl, and butyl chloride, bromides, and iodides; dialkyl sulfates like dimethyl, diethyl, dibutyl, and diamyl sulfates, long chain halides such as decyl, lauryl, myristyl and stearyl chlorides, bromides and iodides, aralkyl halides like benzyl and phenethyl bromides, and others. Water or oil-soluble or dispersible products are thereby obtained.

Examples of acids which may be employed to form pharmaceutically acceptable acid addition salts include such inorganic acids as hydrochloric acid, sulphuric acid and phosphoric acid and such organic acids as oxalic acid, maleic acid, succinic acid and citric acid. Other salts include salts with alkali metals or alkaline earth metals, such as sodium, potassium, calcium or magnesium or with organic bases.

The compounds of the present invention can also be used in the form of prodrugs which include esters. Examples of such esters include a hydroxyl-substituted compound of formula (I) which has been acylated with a blocked or unblocked amino acid residue, a phosphate function, or a hemisuccinate residue. The amino acid esters of particular interest are glycine and lysine; however, other amino acid residues can also be used. Other esters include the compounds of formula (I) wherein a carboxylic acid group has been esterified to provide esters which include, but are not limited to, methyl, ethyl or benzyl esters. These esters serve as prodrugs of the compounds of the present invention and serve to increase the solubility of these substances in the gastrointestinal tract. The prodrugs are metabolically converted in vivo to the parent compound of formula (I). The preparation of the pro-drug esters is carried out by reacting a hydroxyl-substituted compound of formula (I) with an activated amino acyl, phosphoryl or hemisuccinyl derivative. The resulting product is then deprotected to provide the desired prodrug ester. Prodrugs which are esters of carboxylic acid group containing compounds of formula (I) are prepared by methods known in the art.

The novel compounds of the present invention possess an excellent degree of activity and specificity in treating hypertension in a human or other mammal. The novel compounds of the present invention are also useful for treating congestive heart failure in a human or other mammal. The novel compounds of the present invention are also useful for treating vascular abnormalities in a human or other mammal, in particular microvascular diseases associated with diabetes such as diabetic retinopathy, diabetic neuropathy and diabetic neuropathy. In addition, the novel compounds of the invention are useful for treating renal diseases in a human or other mammal, in particular acute and chronic renal failure. The compounds of the invention are also useful for treating psoriasis.

The ability of the compounds of the invention to inhibit human renal renin can be demonstrated in vitro by reacting a selected compound at varied concentrations with human renal renin, free from acid proteolytic

activity, and with renin substrate (human angiotensinogen) at 37 degrees C and pH of 6.0. At the end of the incubation, the amount of angiotensin I formed is measured by radioimmunoassay and the molar concentration required to cause 50% inhibition, expressed as the $IC_{50}$ is calculated. When tested in accordance with the foregoing procedure, the compounds of the invention demonstrated $IC_{50}$'s in the range of $10^{-8}$ to $10^{-10}$M as seen in Table 1.

Table 1

| Example | $IC_{50}$ (nM) |
|---|---|
| 60 | 0.64 |
| 61 | 22 |
| 77 | 1.2 |
| 78 | 2.5 |
| 79 | 1.1 |
| 80 | 1.0 |
| 83 | 1.5 |
| 86 | 1.2 |
| 87 | 5.2 |
| 93 | 1.5 |
| 98 | 0.69 |
| 105 | 0.35 |

The ability of a compound of the invention to decrease blood pressure can be demonstrated according to the following method.

## In Vivo Activity

Intraduodenal activity for the resultant compound from Example 105 was determined at 1.0 mg/kg in male cynomolgus monkeys (*M. fascicularis*) weighing between 3 and 5 kg. Results are shown in Table 2. The monkeys were fed a low-salt chow and fresh fruit diet and treated twice with furosemide (5 mg/kg, po), 1 week and 1 day before the experiment. Following this regimen renders the monkeys in a salt-depleted state, characterized by normal base-line blood pressures but elevated plasma renin activities. The monkeys were fasted overnight and studied under anesthesia on the day of the experiment (sodium pentobarbitol, 15 mg/kg bolus + 0.10 mg/kg/min maintenance iv infusion). A femoral artery was catherterized for the direct and continuous measurement of blood pressure (Grass Pressure Transducer Model P23dB and Graph Polygraph Model 7). The compound was tested for intraduodenal activity via a catheter placed directly into the proximal segment of the duodenum following a laparotomy. Each monkey received only one dose of compound.

Table 2

| Monkey A | | Monkey B | |
|---|---|---|---|
| Time (min) | MBP[a] (mm Hg) | Time (min) | MBP (mm Hg) |
| 0 | 85 | 0 | 89 |
| 5 | 85 | 5 | 88 |
| 10 | 74 | 10 | 76 |
| 15 | 69 | 15 | 79 |
| 20 | 65 | 20 | 75 |
| 25 | 67 | 25 | 74 |
| 30 | 70 | 30 | 75 |
| 35 | 71 | 35 | 78 |
| 40 | 74 | 40 | 83 |
| 45 | 74 | 44 | 83 |
| 50 | 76 | 49 | 82 |
| 55 | 77 | 54 | 83 |
| 60 | 79 | 59 | 86 |
| 65 | 79 | 64 | 83 |
| 70 | 79 | 69 | 86 |
| 75 | 80 | 74 | 87 |
| 80 | 79 | 79 | 89 |
| 85 | 80 | 84 | 87 |
| 90 | 80 | 89 | 92 |
| 95 | 81 | 94 | 86 |
| 100 | 82 | 99 | 90 |
| 105 | 83 | 104 | 91 |
| 109 | 81 | 109 | 90 |
| 114 | 81 | 114 | 92 |
| 119 | 81 | 119 | 92 |

[a] Mean blood pressure.

Total daily dose administered to a host in single or divided doses may be in amounts, for example, from 0.001 to 10 mg/kg body weight daily and more usually 0.01 to 10 mg. Dosage unit compositions may contain such amounts of submultiples thereof to make up the daily dose.

The amount of active ingredient that may be combined with the carrier materials to produce a single dosage form will vary depending upon the host treated and the particular mode of administration.

It will be understood, however, that the specific dose level for any particular patient will depend upon a variety of factors including the activity of the specific compound employed, the age, body weight, general health, sex, diet, time of administration, route of administration, rate of excretion, drug combination, and the severity of the particular disease undergoing therapy.

The compounds of the present invention may be administered orally, parenterally, by inhalation spray, rectally, or topically in dosage unit formulations containing conventional nontoxic pharmaceutically acceptable carriers, adjuvants, and vehicles as desired. Topical administration may also involve the use of transdermal administration such as transdermal patches or iontophoresis devices. The term parenteral as used herein includes subcutaneous injections, intravenous, intramuscular, intrasternal injection, or infusion techniques.

Injectable preparations, for example, sterile injectable aqueous or oleagenous suspensions may be formulated according to the known art using suitable dispersing or wetting agents and suspending agents. The sterile injectable preparation may also be a sterile injectable solution or suspension in a nontoxic parenterally acceptable diluent or solvent, for example, as a solution in 1,3-butanediol. Among the acceptable vehicles and solvents that may be employed are water, Ringer's solution, and isotonic sodium chloride solution. In addition, sterile, fixed oils are conventinally employed as a solvent or suspending medium. For this purpose any bland fixed oil may be employed including synthetic mono- or diglycerides. In addition, fatty acids such as oleic acid find use in the preparation of injectables.

Suppositories for rectal administration of the drug can be prepared by mixing the drug with a suitable nonirritating excipient such as cocoa butter and polyethylene glycols which are solid at ordinary temperatures but liquid at the rectal temperature and will therefore melt in the rectum and release the drug.

Solid dosage forms for oral administration may include capsules, tablets, pills, powders, and granules. In such solid dosage forms, the active compound may be admixed with at least one inert diluent such as sucrose lactose or starch. Such dosage forms may also comprise, as is normal practice, additional substances other than inert diluents, e.g., lubricating agents such as magnesium stearate. In the case of capsules, tablets, and pills, the dosage forms may also comprise buffering agents. Tablets and pills can additionally be prepared with enteric coatings.

Liquid dosage forms for oral administration may include pharmaceutically acceptable emulsions, solutions, suspensions, syrups, and elixirs containing inert diluents commonly used in the art, such as water. Such compositions may also comprise adjuvants, such as wetting agents, emulsifying and suspending agents, and sweetening, flavoring, and perfuming agents.

The present invention also relates to the use of novel compounds, pharmaceutical compositions containing the novel compounds and the use of the compounds and compositions to inhibit renin for treating glaucoma or reducing and/or controlling intraocular pressure. The present invention also relates to the use of novel compounds and pharmaceutical compositions which inhibit renin in combination with a beta-adrenergic antagonist agent or an angiotensin converting enzyme inhibiting compound for treating glaucoma or reducing and/or controlling intraocular pressure.

The present invention also relates to pharmaceutical compositions for treating the increase in intraocular pressure associated with the administration of steroidal antiinflammatory agents comprising novel renin inhibiting compounds in combination with a steroidal antiinflammatory compound in a pharmaceutically acceptable vehicle.

The present invention also relates to a kit comprising in individual containers in a single package a novel renin inhibiting compound in a suitable pharmaceutical vehicle and a steroidal antiinflammatory compound in a suitable pharmaceutical vehicle and/or a beta-adrenergic antagonist agent in a suitable pharmaceutical vehicle or an angiotensin converting enzyme inhibiting compound in a suitable pharmaceutical vehicle.

The compositions of the invention are administered as topical or systemic pharmaceutical compositions when used for treating or reducing and/or controlling intraocular pressure.

These compositions are preferably administered as topical pharmaceutical compositions suitable for ophthalmic administration, in a pharmaceutically acceptable vehicle such as pharmaceutically acceptable sterile aqueous or nonaqueous solutions, suspensions, emulsions, ointments and solid inserts.

Examples of suitable pharmaceutically acceptable vehicles for ophthalmic administration are water, propylene glycol and other pharmaceutically acceptable alcohols, sesame or peanut oil and other pharmaceutically acceptable vegetable oils, petroleum jelly, water soluble ophthalmologically acceptable nontoxic polymers such as methyl cellulose, carboxymethyl cellulose salts, hydroxyethyl cellulose, hydroxypropyl cellulose; acrylates such as polyacrylic acid salts; ethylacrylates; polyacrylamides; natural products such as gelatin, alginates, pectins, tragacanth, karaya, agar, acacia; starch derivatives such as starch acetate, hydroxyethyl starch ethers, hydroxypropyl starch; as well as other synthetic derivatives such as polyvinyl alcohol, polyvinyl pyrrolidone, polyvinyl methyl ether, polyethylene oxide, carbopol and xantham gum; and mixtures of these polymers. Such compositions may also contain adjuvants such as buffering, preserving, wetting, emulsifying, and dispersing agents. Suitable preserving agents include antibacterial agents such as quaternary ammonium compounds, phenylmercuric salts, benzyl alcohol, phenyl ethanol; and antioxidants such as sodium metabisulfite, butylated hydroxyanisole and butylated hydroxytoluene. Suitable buffering agents include borate, acetate, gluconate and phosphate buffers.

The pharmaceutical ophthalmic compositions of the invention may also be in the form of a solid insert. A solid water soluble or water swellable polymer such as dextran, hydroxyloweralkyl dextran, carboxymethyl dextran, hydroxyloweralkyl cellulose, loweralkyl cellulose, carboxymethyl cellulose, polyvinyl alcohol, dextrin, starch, polyvinyl pyrrolidone and polyalkylene glycols may be used as the carrier for the drug.

Dosage levels of the active compound in the compositions for treating glaucoma or reducing and/or controlling intraocular pressure may be varied so as to obtain a desired therapeutic response to a particular, composition. Generally, the active compound will be administered as an isotonic aqueous solution of from 0.00001 to 1.0 (w/v) percent concentration. More preferably the active compound will be administered as an isotonic aqueous solution of from 0.00001 to 0.1 (w/v) percent concentration.

The term "controlling intraocular pressure" as used herein means the regulation, attenuation and modulation of increased intraocular tension. The term also means that the decrease, in the otherwise elevated intraocular pressure, obtained by the methods and compositions of the invention is maintained for

a significant period of time as, for example, between consecutive doses of the composition of the invention.

The novel renin inhibiting compounds of the invention may be the only active ingredient for controlling intraocular pressure in the methods and compositions of the invention or may be used in combination with other ingredients which control intraocular pressure such as beta-adrenergic antagonist compounds. The term "beta-adrenergic antagonist" as used herein means a compound which by binding to betaadrenergic plasma membrane receptors reduces or eliminates sympathetic activity or blocks the effects of exogenously adminstered catecholamines or adrenergic drugs. Examples of beta-adrenergic antagonists are atenolol, metopropol, nadolol, propranolol, timolol, labetalol, betaxolol, carteolol and dilevalol and pharmaceutically acceptable salts thereof. Most preferably the beta-adrenergic antagonist is timolol.

Timolol is currently used for treating glaucoma or reducing and/or controlling intraocular pressure, but it has a number of adverse side effects. Accordingly, administration of a composition comprising a combination of a beta-adrenergic antagonist and a novel renin inhibiting compound of the invention could produce a reduction in intraocular pressure equivalent to that produced by a beta-adrenergic antagonist alone, but at a reduced dose level of the beta-adrenergic antagonist. This will result in a reduced level of the beta-adrenergic antagonist related adverse side effects.

The combination composition is administered as a single dosage form containing both the novel renin inhibitor and the beta-adrenergic antagonist. The beta adrenergic antagonist may comprise from 5 mg to about 125 mg of the composition of the invention. The preferred ranges of the components in the composition of the invention in unit dosage form are:

Renin inhibitor: 1 ng to 0.1 mg

Beta-adrenergic antagonist: 5 ug to 125 ug

When the beta-adrenergic antagonist and the novel renin inhibitor are administered as separate compositions the present invention relates to a kit comprising in two separate containers a pharmaceutically acceptable beta-adrenergic antagonist composition and a pharmaceutically acceptable novel renin inhibitor composition, in a single package. A preferred kit comprises a beta-adrenergic antagonist composition and a topical novel renin inhibitor composition. A most preferred kit comprises a topical ophthalmological beta-adrenergic antagonist composition and a topical ophthalmological novel renin inhibitor composition.

The novel renin inhibiting compounds of the invention may also be administered in combination with an angiotensin converting enzyme (ACE) inhibiting compound. Examples of angiotensin converting enzyme inhibiting compounds are captopril and enalapril. As was previously mentioned, ACE inhibitors have some undesirable side effects. Accordingly, administration of an ACE inhibitor in combination with a renin inhibitor could produce a reduction in intraocular pressure greater than or equivalent to that of an ACE inhibitor alone, but at a reduced dose level of the ACE inhibitor. This will result in a reduced level of the ACE inhibitor related adverse side effects.

The combination composition is administered as a single dose form containing both the novel renin inhibitor and the angiotensin converting enzyme inhibitor. The ACE inhibitor may comprise from 5 ng to about 50 ug of the compositon of the invention. The preferred ranges of the components in the composition of the invention in unit dosage form are:

Renin inhibitor: 1 ng to 0.1 mg

ACE inhibitor: 5 ng to 50 ug

When the ACE inhibitor and the novel renin inhibitor are administered as separate compositions the present invention relates to a kit comprising in two separate containers a pharmaceutically acceptable ACE inhibitor composition and a pharmaceutically acceptable novel renin inhibitor composition, in a single package. A preferred kit comprises an ACE inhibitor composition and a topical novel renin inhibitor composition. A most preferred kit comprises a topical ophthalmological ACE inhibitor composition and a topical novel renin inhibitor composition.

Dosage levels of the active compounds in the compositions of the invention may be varied so as to obtain a desired therapeutic response depending on the route of administration, severity of the disease and the response of the patient.

Topical, ophthalmic and systemic administration of steroidal antiinflammatory agents can cause an increase in intraocular pressure. The increase in intraocular pressure can be reduced by the administration of a novel renin inhibiting compound of the invention. Steroidal antiinflammatory agents include hydrocortisone, cortisone, prednisone, prednisolone, dexamethasone, methylprednisolone, triamcinolone, betamethasone, alclometasone, flunisolide, beclomethasone, clorocortolone, diflorasone, halcinonide, fluocinonide, fluocinolone, desoximetasone, medrysone, paramethasone, and fluorometholone, and their pharmaceutically acceptable salts and esters. Preferred steroidal antiinflammatory agents are hydrocortisone, prednisolone, dexamethasone, medrysone and fluorometholone and their pharmaceutically acceptable salts and esters. The novel renin inhibitor is administered after use of a steroidal antiinflammatory

agent or at the same time, causing reduction and/or control of intraocular pressure.

Various combinations of a topical or oral or injectible dosage form of a steroidal antiinflammatory agent and a topical or oral dosage form of the novel renin inhibitor may be used. A preferred combination comprises a topical steroidal antiinflammatory and a topical novel renin inhibitor. More preferred is a topical ophthalmic dosage form comprising both a steroidal antiinflammatory and a novel renin inhibitor.

When the steroidal antiinflammatory agent and the novel renin inhibitor are administered as separate compositions the present invention relates to a kit comprising in two separate containers a pharmaceutically acceptable steroidal antiinflammatory agent composition and a pharmaceutically acceptable novel renin inhibitor composition, in a single package. A preferred kit comprises a steroidal antiinflammatory composition and a topical novel renin inhibitor composition. A most preferred kit comprises a topical ophthamological steroidal antiinflammatory composition and a topical ophthamological novel renin inhibitor composition.

The combination composition of the invention may contain from about 0.00001 to 1.0 (w/v) percent of the novel renin inhibitor for combined or separate topical administration. More preferably the amount of the novel renin inhibitor is about 0.00001 to 0.1 (w/v) percent of the composition. The amount of the novel renin inhibitor in a unit dosage form for topical administration to the eye is from about 5 ng to about 0.5 mg, preferably from about 5 ng to about 25 ng. The dose required will depend on the potency of the particular novel renin inhibitor, the severity of the intraocular pressure increase and the response of the individual patient.

The combination composition of the invention may contain from about 0.05 to 1.5 (w/v) percent of the steroidal antiinflammatory for combined or separate topical administration. The amount of the steroidal antiinflammatory in a unit dosage form for topical administration to the eye is from about 20 ug to about 600 ug. The dose required will depend on the potency of the particular steroidal antiinflammatory, the severity of the disease and the response of the individual patient.

When the steroidal antiinflammatory agent of the combination therapeutic method of the invention is administered other than ophthalmically, appropriate doses are well known in the art.

The compositions of the invention may include other therapeutic agents in addition to the novel renin inhibitor, and other agents which reduce and/or control intraocular pressure.

The effect on intraocular pressure of the novel compounds of the invention can be determined in rabbits by using the following method.

## Effects of Topically Administered Renin Inhibiting Compounds on Intraocular Pressure of Rabbits

### a. Method

The antiglaucoma activity of the compounds was tested by measuring the effect on intraocular pressure in rabbits as described by Tinjum, A.M., Acta Ophthalmologica, 50 , 677 (1972). Male albino, New Zealand rabbits were placed in restraining devices and the intraocular pressure was measured with an applamatic tonometer. Exactly 0.1 ml of an isotonic saline solution containing a test compound was instilled into the conjuctival sac and the intraocular pressure was measured at 5, 15, 30, 60, 90, 120 and 180 minutes afterwards.

The ability of a compound of the invention to treat vascular diseases, especially those associated with diabetes, can be demonstrated by comparing urinary protein excretion in control diabetic Wistar rats with urinary protein excretion in diabetic Wistar rats treated with a compound of the invention. Wistar rats are made diabetic by streptozocin treatment.

The present invention is also directed to the use of compounds of the formula I in combination with one or more antihypertensive agents independently selected from diuretics, adrenergic blocking agents, vasodilators, calcium channel blockers, angiotensin converting enzyme (ACE) inhibitors, potassium channel activators and other antihypertensive agents for treating (in a human or other mammal) hypertension, congestive heart failure, vascular disease related to diabetes or for treating renal diseases such as acute or chronic renal failure.

Representative diuretics include hydrochlorothiazide, chlorothiazide, acetazolamide, amiloride, bumetanide, benzthiazide, ethacrynic acid, furosemide, indacrinone, metolazone, spironolactone, triamterene, chlorthalidone and the like or a pharmaceutically acceptable salt thereof.

Representative adrenergic blocking agents include phentolamine, phenoxybenzamine, prazosin,

terazosin, tolazine, atenolol, metoprolol, nadolol, propranolol, timolol, carteolol and the like or a pharmaceutically acceptable salt thereof.

Representative vasodilators include hydralazine, minoxidil, diazoxide, nitroprusside and the like or a pharmaceutically acceptable salt thereof.

Representative calcium channel blockers include amrinone, bencyclane, diltiazem, fendiline, flunarizine, nicardipine, nimodipine, perhexilene, verapamil, gallopamil, nifedipine and the like or a pharmaceutically acceptable salt thereof.

Representative ACE inhibitors include captopril, enalapril, lisinopril and the like or a pharmaceutically acceptable salt thereof.

Representative potassium channel activators include pinacidil and the like or a pharmaceutically acceptable salt thereof.

Other representative antihypertensive agents include sympatholytic agents such as methyldopa, clonidine, guanabenz, reserpine and the like or a pharmaceutically acceptable salt thereof.

The compound of formula I and the antihypertensive agent can be administered at the recommended maximum clinical dosage or at lower doses. Dosage levels of the active compounds in the compositions of the invention may be varied so as to obtain a desired therapeutic response depending on the route of administration, severity of the disease and the response of the patient. The combination can be administered as separate compositions or as a single dosage form containing both agents.

In addition, the present invention is directed to the use of a compound of formula I to inhibit retroviral proteases and in particular to inhibit HIV-1 protease and HIV-2 protease. Compounds of formula I are useful for treatment or prophylaxis of diseases caused by retroviruses, especially acquired immune deficiency syndrome or an HIV infection.

The inhibitory potency of the compounds of the invention against HIV protease can be determined by the following method.

<u>Fluorogenic Assay for Screening Inhibitors of HIV Protease</u>

A compound of the invention is dissolved in DMSO and a small aliquot further diluted with DMSO to 100 times the final concentration desired for testing. The reaction is carried out in a 6 X 50 mm tube in a total volume of 300 microliters. The final concentrations of the components in the reaction buffer are: 125 mM sodium acetate, 1 M sodium chloride, 5 mM dithiothreitol, 0.5 mg/ml bovine serum albumin, 1.3 uM fluorogenic substrate, 2% (v/v) dimethylsulfoxide, pH 4.5. After addition of inhibitor, the reaction mixture is placed in the fluorometer cell holder and incubated at 30° C for several minutes. The reaction is initiated by the addition of a small aliquot of cold HIV protease. The fluorescence intensity (excitation 340 nM, emmision 490 nM) is recorded as a function of time. The reaction rate is determined for the first six to eight minutes. The observed rate is directly proportional to the moles of substrate cleaved per unit time. The percent inhibition is 100 X (1 - (rate in presence of inhibitor)/(rate in absence of inhibitor)).

Fluorogenic substrate: Dabcyl-Ser-Gln-Asp-Tyr-Pro-Ile-Val-Gln-EDANS wherein DABCYL = 4-(4-dimethylaminophenyl)azobenzoic acid and EDANS = 5-((2-aminoethyl)amino)-naphthalene-1-sulfonic acid.

The antiviral activity of compounds of the invention can be demonstrated using the following method.

A mixture of 0.1 ml (4 X $10^6$ cells/ml) of H9 cells and 0.1 ml (100 infectious units) of HIV-$1_{3B}$ was incubated on a shaker for 2 h. The resulting culture was washed three times, resuspended into 2 ml of medium, and treated with 10 $\mu$l of the compound of the invention (5 mM in dimethylsulfoxide). The control culture was treated in an identical manner except the last step was omitted. After incubation of the culture for eight days without change of medium, an aliquot (0.1 ml) of the supernatent was withdrawn and incubated with fresh H9 cells on a shaker for 2 h. The resulting culture was washed three times, resuspended into 2 ml of medium, and incubated. Virus infectivity was determined using the Abbott HTLV-III antigen E.I.A. method (Paul, et al., J. Med. Virol., <u>22</u> 357 (1987)).

The foregoing is merely illustrative of the invention and is not intended to limit the invention to the disclosed compounds. Variations and changes which are obvious to one skilled in the art are intended to be within the scope and nature of the invention which are defined in the appended claims.

**Claims**

1. A compound of the formula:

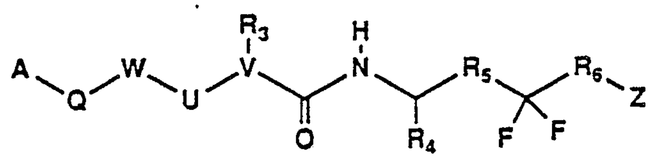

wherein A is hydrogen, loweralkyl, functionalized alkyl, aryl, heterocyclic, $-OR_7$ or $-SR_7$ wherein $R_7$ is hydrogen, loweralkyl, functionalized alkyl, aryl, heterocyclic, aryl-C(O)- or heterocyclic-C(O)-, carboxy, alkoxycarbonyl, functionalized amino, functionalized hydroxyalkyl or substituted carbonyloxy or substituted carbonyloxy analog;

W is -C(O)-, -CH(OH)- or $-N(R_2)-$ wherein $R_2$ is hydrogen or loweralkyl;

U is -C(O)-, $-CH_2-$ or $-N(R_2)-$ wherein $R_2$ is hydrogen or loweralkyl, with the proviso that when W is -CH-(OH)-then U is $-CH_2-$ and with the proviso that U is -C(O)- or $-CH_2-$ when W is $-N(R_2)-$;

V is -CH-, -C(OH)- or -C(halogen)- with the proviso that V is -CH- when U is $-N(R_2)-$;

Q is $-CH(R_1)-$ or $-C(=CHR_{1a})-$ wherein $R_1$ is loweralkyl, cycloalkylalkyl, arylalkyl, (heterocyclic)alkyl, 1-benzyloxyethyl, phenoxy, thiophenoxy or anilino, provided that B is $-CH_2-$ or -CH(OH)- or A is hydrogen when $R_1$ is phenoxy, thiophenoxy or anilino and $R_{1a}$ is aryl or heterocyclic;

$R_3$ is loweralkyl, loweralkenyl, hydroxyalkyl, alkoxyalkyl, ((alkoxy)alkoxy)alkyl, carboxyalkyl, (thioalkoxy)alkyl, aziodoalkyl, aminoalkyl, (alkyl)aminoalkyl, (dialkyl)aminoalkyl, (alkoxy) (alkyl)aminoalkyl, (alkoxy)aminoalkyl, benzyl or (heterocyclic)methyl;

$R_4$ is loweralkyl, cycloalkylmethyl or benzyl;

$R_5$ is -CH(OH)- or -C(O)-;

$R_6$ is -CH(OH)- or -C(O)-; and

Z is lower alkyl, aryl, arylalkyl, cycloalkyl, cycloalkylalkyl, heterocyclic or (heterocyclic)alkyl;

or a pharmaceutically acceptable salt, ester or prodrug thereof.

2. The compound of Claim 1 wherein A is alkoxycarbonylamino or $R_{10}-C(O)-CH_2-$ wherein $R_{10}$ is (-(heterocyclic)alkyl) (alkyl)amino or heterocyclic; $R_1$ is benzyl or 4-methoxybenzyl; W is -C(O)-; U is -NH-; V is -CH-; $R_3$ is (heterocyclic)methyl; $R_4$ is isobutyl or cyclohexylmethyl; and Z is loweralkyl or cycloalkyl.

3. A compound of the formula:

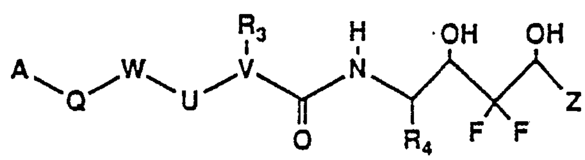

wherein A is

A is

(1) hydrogen,

(2) loweralkyl,

(3) functionalized alkyl,

(4) aryl,

(5) heterocyclic,

(6) $-OR_7$ or $-SR_7$ wherein $R_7$ is

(i) hydrogen,

(ii) loweralkyl,

(iii) functionalized alkyl,

(iv) aryl,

(v) heterocyclic,

(vi) aryl-C(O)- or

(vii) heterocyclic-C(O)-,

(7) carboxy,

(8) alkoxycarbonyl,

(9) functionalized amino,

(10) functionalized hydroxyalkyl or

(11) substituted carbonyloxy or substituted carbonyloxy analog;

W is
(1) -C(O)-,
(2) -CH(OH)- or
(3) -N($R_2$)- wherein $R_2$ is hydrogen or loweralkyl;
U is
(1) -C(O)-,
(2) -$CH_2$- or
(3) -N($R_2$)- wherein $R_2$ is hydrogen or lower alkyl, with the proviso that when W is -CH(OH)-then U is -$CH_2$- and with the proviso that U is -C(O)- or -$CH_2$- when W is -N($R_2$)-;
V is
(1) -CH-,
(2) -C(OH)- or
(3) -C(halogen)- with the proviso that V is -CH-when U is -N($R_2$)-;
Q is -CH($R_1$)- or -C(=CH$R_{1a}$)- wherein $R_1$ is
(1) loweralkyl,
(2) cycloalkylalkyl,
(3) arylalkyl,
(4) (heterocyclic)alkyl,
(5) 1-benzyloxyethyl,
(6) phenoxy,
(7) thiophenoxy or
(8) anilino, provided that B is -$CH_2$- or -CH(OH)-or A is hydrogen when $R_1$ is phenoxy, thiophenoxy or anilino and $R_{1a}$ is aryl or heterocyclic;
$R_3$ is
(1) loweralkyl,
(2) loweralkenyl,
(3) hydroxyalkyl,
(4) alkoxyalkyl,
(5) ((alkoxy)alkoxy)alkyl,
(6) carboxyalkyl,
(7) (thioalkoxy)alkyl,
(8) azidoalkyl,
(9) aminoalkyl,
(10) (alkyl)aminoalkyl,
(11) (dialkyl)aminoalkyl,
(12) (alkoxy)(alkyl)aminoalkyl,
(13) (alkoxy)aminoalkyl,
(14) benzyl or
(15) (heterocyclic)methyl
; $R_4$ is
(1) loweralkyl,
(2) cycloalkylmethyl or
(3) benzyl; and
Z is
(1) lower alkyl,
(2) aryl,
(3) arylalkyl,
(4) cycloalkyl,
(5) cycloalkylalkyl,
(6) heterocyclic or
(7) (heterocyclic)alkyl;
or a pharmaceutically acceptable salt, ester or prodrug thereof.

4. The compound of Claim 3 wherein A is alkoxycarbonylamino or $R_{10}$-C(O)-$CH_2$- wherein $R_{10}$ is (-(heterocyclic)alkyl)(alkyl)amino or heterocyclic; $R_1$ is benzyl or 4-methoxybenzyl; W is -C(O)-; U is -NH-; V is -CH-; $R_3$ is (heterocyclic)methyl; $R_4$ is isobutyl or cyclohexylmethyl; and Z is loweralkyl or cycloalkyl.

5. A compound selected from the group consisting of:
(2R)-2-Benzyl-3-((2-pyrazol-1-ylethyl)methylaminocarbonyl)propionic acid amide of L-(4-thiazolyl)alanine-2-(S)-amino-1-cyclohexyl-4,4-difluoro-3(R),5(R)-dihydroxy-6-methylheptane;

2(R)-2-Benzyl-3-Morpholinocarbonylpropionic acid amide of His-2(S)-Amino-1-cyclohexyl-4,4-difluoro-3(R),5-(R)-dihydrcxy-6-methylheptane;

(E)-2-((4-Morpholinylcarbonyl)methyl)cinnamic acid amide of His-2(S)-amino-1-cyclohexyl-4,4-difluoro-3(R)-,5(R)-dihydroxy-6-methylheptane;

2(R)-2-Benzyl-3-((2-imidazolylethyl)methylaminocarbonyl)propionic acid amide of L-(4-thiazolyl]alanine-2(S)-amino-1-cyclohexyl-4,4-difluoro-3(R),5(R)-dihydroxy-6-methylheptane;

Boc-Phe-Leu amide of 2(S)-Amino-1-cyclohexyl-4,4-difluoro-3,5-dioxo-6-methylheptane; and

2(R)-2-Benzyl-3(4-methypiperazine-1-ylcarbonyl)propionic acid amide of L-(4-thiazolyl)alamine-2(S)-amino-1-cyclohexyl-4,4-difluoro-3(R),5(R)-dihydroxy-6-methylheptane; or a pharmaceutically acceptable salt, ester or prodrug thereof.

6. A compound according to any one of claims 1 to 5 for use in inhibiting renin in a mammal in need of such treatment.

7. A pharmaceutical composition for inhititing renin comprising a pharmaceutical carrier and a therapeutically effective amount of a compound of Claim 1.

8. A compound according to any one of Claims 1 to 5 alone or in combination with another antihypertensive agent for use in treating hypertension or congestive heart failure in a mammal in need of such treatment.

9. A pharmaceutical composition for treating hypertension or congestive heart failure, comprising a pharmaceutical carrier and a therapeutically effective amount of a compound of Claim 1 alone or in combination with another antihypertensive agent.

10. A compound according to any one of Claims 1 to 5 for use in disease, renal failure, psoriasis or a retroviral infection or for inhibiting retroviral proteases in a mammal in need of such treatment.

11. A compound of the formula:

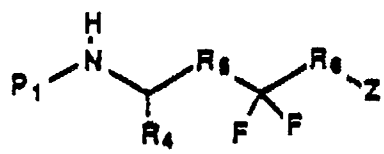

wherein

$P_1$ is hydrogen or an N-protecting group.

$P_1$ is hydrogen or an N-protecting group;

$R_4$ is

(1) loweralkyl,

(2) cycloalkylmethyl or

(3) benzyl;

$R_5$ is

(1) -CH(OH)- or

(2) -C(O)-;

$R_6$ is

(1) -CH(OH)- or

(2) -C(O)-; and

Z is

(1) lower alkyl,

(2) aryl,

(3) arylalkyl,

(4) cycloalkyl,

(5) cycloalkylalkyl,

(6) heterocyclic or

(7) (heterocyclic)alkyl.

12. A process for the preparation of a compound according to any one of Claims 1 to 5 comprising reacting a compound of the formula:

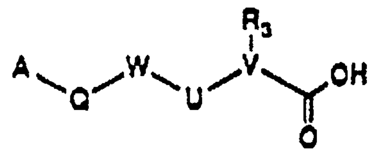

or an activated ester thereof with an amine of the formula:

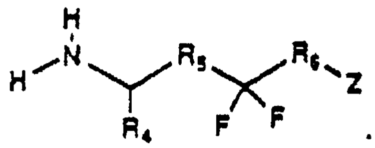

European Patent Office

# EUROPEAN SEARCH REPORT

Application Number

**EP 90 11 6225**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | EP-A-0 202 577 (G.D. SEARLE & CO.)<br>* Page 5, line 10; page 12, scheme 2 - page 14, example 4; claims 1,3,4,9-11 * | 1-12 | C 07 K 5/06<br>C 07 D 233/64<br>C 07 D 277/30<br>A 61 K 31/415<br>A 61 K 31/54<br>A 61 K 37/64 |
| Y | J. MED. CHEM., vol. 29, 1986, pages 2080-2087, American Chemical Society; S. THAISRIVONGS et al.: "Design and synthesis of potent and specific renin inhibitors containing difluorostatine, difluorostatone, and related analogues"<br>* Entire document * | 1-12 | |
| X | WO-A-8 704 349 (J. DELLARIA & CO.)<br>* Examples 86-105,312-315; claims 1,2,6,7,13,14, especially example 87 * | 11,12 | |
| A | | 1-10 | |
| A | EP-A-0 274 259 (SANKYO CO., LTD)<br>* Entire document * | 1-10 | |
| P,X | EP-A-0 368 719 (MERREL DOW PHARM.)<br>* Example 35; claims 1-4,6-8 * | 11,12 | |
| | | | TECHNICAL FIELDS SEARCHED (Int. Cl.5) |
| | | | C 07 K<br>C 07 D<br>A 61 K |

The present search report has been drawn up for all claims

| Place of search | Date of completion of search | Examiner |
|---|---|---|
| The Hague | 03 December 90 | GROENENDIJK M.S.M. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention

E: earlier patent document, but published on, or after the filing date
D: document cited in the application
L: document cited for other reasons

&: member of the same patent family, corresponding document